# EUROPÄISCHE PATENTSCHRIFT

(11) **EP 1 276 741 B1**
(45) Veröffentlichungstag und Bekanntmachung des Hinweises auf die Patenterteilung: **31.12.2008**
(21) Anmeldenummer: 01940288.2
(22) Anmeldetag: 05.04.2001
(51) Int. Cl.: C07D 455/02, A01N 43/90, A01N 43/40, C07D 409/10, C07D 221/20, A01N 43/42, C07D 491/10, C07D 211/86, C07D 211/34, C07C 233/51, C07D 309/08, C07D 309/14, C07C 233/52

(54) **PHENYLSUBSTITUIERTE 4-HYDROXY-TETRAHYDROPYRIDONE UND IHRE VERWENDUNG ALS SCHÄDLINGSBEKÄMPUNGSMITTEL UND HERBIZIDE**
PHENYL SUBSTITUTED 4-HYDROXY TETRAHYDROPYRIDONE AND ITS USE AS PESTICIDE AND HERBICIDE
4-HYDROXYTETRAHYDROPYRIDONE A SUBSTITUTION PHENYLE ET LEUR UTILISATION EN TANT QUE PESTICIDES ET HERBICIDES

(30) Priorität: 18.04.2000 DE 10019145
(43) Veröffentlichungstag der Anmeldung: 22.01.2003
(73) Patentinhaber: Bayer CropScience AG, 40789 Monheim (DE)
(72) Erfinder: FISCHER, Reiner, 40789 Monheim (DE); GRAFF, Alan, 51375 Leverkusen (DE); TRAUTWEIN, Axel, 51467 Bergisch Gladbach (DE); ULLMANN, Astrid, 50677 Köln (DE); SCHNEIDER, Udo, 51373 Leverkusen (DE); WISCHNAT, Ralf, 51061 Köln (DE); DREWES, Mark, Wilhelm, 40764 Langenfeld (DE); ERDELEN, Christoph, 42799 Leichlingen (DE); FEUCHT, Dieter, 40789 Monheim (DE)
(86) Internationale Anmeldenummer: PCT/EP2001/003864
(87) Internationale Veröffentlichungsnummer: WO 2001/079204

(56) Entgegenhaltungen:
- WO-A-00/41469
- WO-A-01/17972
- DE-A- 19 543 864
- PATENT ABSTRACTS OF JAPAN vol. 1999, no. 11, 30. September 1999 (1999-09-30) -& JP 11 152273 A (OTSUKA CHEM CO LTD), 8. Juni 1999 (1999-06-08) in der Anmeldung erwähnt
- DATABASE CHEMABS [Online] CHEMICAL ABSTRACTS SERVICE, COLUMBUS, OHIO, US; TAKAHASHI, HIROTAKE ET AL: "Agricultural and horticultural herbicides containing N-[(halophenyl) alkanoyl]amino acid derivatives" retrieved from STN Database accession no. 82:27233 XP002174236 & JP 49 011415 B (NISSAN CHEMICALS INDUSTRIES, LTD.) 16. März 1974 (1974-03-16)

## Beschreibung

Die vorliegende Erfindung betrifft neue phenylsubstituierte 4-Hydroxy-tetrahydropyridone, mehrere Verfahren zu ihrer Herstellung und ihre Verwendung als Schädlingsbekämpfungsmittel und Herbizide.

Es ist bekannt, dass bestimmte Tetrahydropyridone herbizide Eigenschaften besitzen: JP 0832530. Außerdem sind spezielle 4-Hydroxytetrahydropyridone mit akariziden, insektiziden und herbiziden Eigenschaften bekannt: JP 11152273.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 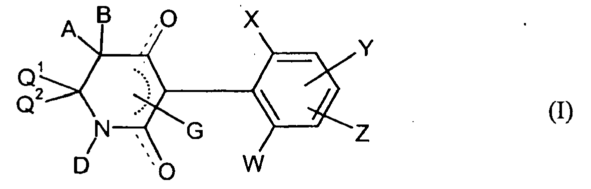 in welcher
- W: steht insbesondere bevorzugt für Wasserstoff, Fluor, Chlor, Brom, Methyl oder Ethyl,
- X: steht insbesondere bevorzugt für Chlor, Brom, Methyl, Ethyl, Propyl oder Trifluormethyl,
- Y: steht insbesondere bevorzugt für Wasserstoff, Methyl, Ethyl, Fluor, Chlor, Brom oder für den Rest 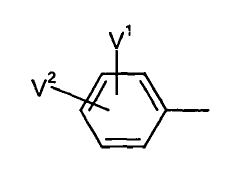
V¹ steht insbesondere bevorzugt für Wasserstoff, Fluor, Chlor, Brom, Methyl, tert.-Butyl, Methoxy, Trifluormethyl oder Phenyl,
V² steht insbesondere bevorzugt für Wasserstoff, Fluor, Chlor, Methyl, Methoxy oder Trifluormethyl,
- Z: steht insbesondere bevorzugt für Wasserstoff, Chlor, Brom oder Methyl,
- A und B: stehen insbesondere bevorzugt für Wasserstoff,
- D: steht insbesondere bevorzugt für Wasserstoff, Methyl, Ethyl, Propyl, iso-Propyl, Cyclopropyl, Cyclopentyl oder Cyclohexyl,
- D und Q¹: stehen gemeinsam insbesondere bevorzugt für Butandiyl,
- Q¹: steht insbesondere bevorzugt für Methyl, Ethyl oder Propyl,
- Q²: steht insbesondere bevorzugt für Methyl, wenn D und Q¹ gemeinsam für Butandiyl stehen, kann Q² auch für Wasserstoff stehen,
- Q¹ und Q²: stehen insbesondere bevorzugt gemeinsam mit dem Kohlenstoff, an den sie gebunden sind für gegebenenfalls durch Methyl, Ethyl, Methoxy, Ethoxy, Propoxy oder Butoxy substituiertes C₅-C₆-Cycloalkyl, in welchem gegebe- nenfalls ein Ringglied durch Sauerstoff ersetzt ist.
- G: steht insbesondere bevorzugt für Wasserstoff (a) oder für eine der Gruppen 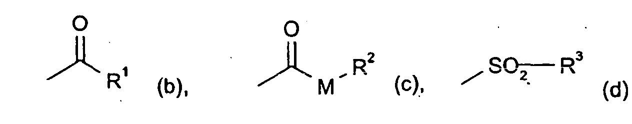 oder 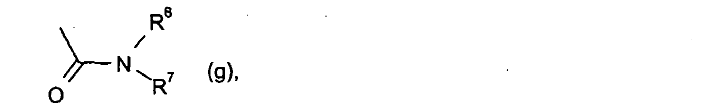 worin M für Sauerstoff oder Schwefel steht,
- R¹: steht insbesondere bevorzugt für C₁-C₈-Alkyl, C₂-C₄-Alkenyl, Methoxy- methyl, Ethoxymethyl, Ethylthiomethyl oder gegebenenfalls durch Fluor, Chlor, Methyl, Ethyl oder Methoxy substituiertes Cyclopropyl, Cyclopentyl oder Cyclohexyl, für gegebenenfalls durch Fluor, Chlor, Brom, Cyano, Nitro, Methyl, Ethyl, iso-Propyl, tert-Butyl, Methoxy, Trifluormethyl oder Trifluormethoxy substi- tuiertes Phenyl, für jeweils gegebenenfalls durch Chlor oder Methyl substituiertes Thienyl oder Pyridyl,
- R²: steht insbesondere bevorzugt für jeweils gegebenenfalls durch Fluor sub- stituiertes C₁-C₈-Alkyl, C₂-C₄-Alkenyl oder Methoxyethyl, Ethoxyethyl, Propoxyethyl, iso-Propoxyethyl, Cyclopentyl oder Cyclohexyl, oder für jeweils gegebenenfalls durch Fluor, Chlor, Cyano, Nitro, Methyl, Ethyl, iso-Propyl, tert-Butyl, Methoxy, Trifluormethyl oder Trifluormethoxy substituiertes Phenyl oder Benzyl.
- R³: steht insbesondere bevorzugt für Methyl.
- R⁶ und R⁷: stehen insbesondere bevorzugt zusammen für einen C₆-Alkylenrest, in welchem gegebenenfalls eine Methylengruppe durch Sauerstoff ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sein können.

Die Verbindungen der Formel (I) können in Abhängigkeit von der Stellung des Substituenten G in den zwei isomeren Formen der Formeln (I-A) und (I-B) vorliegen, 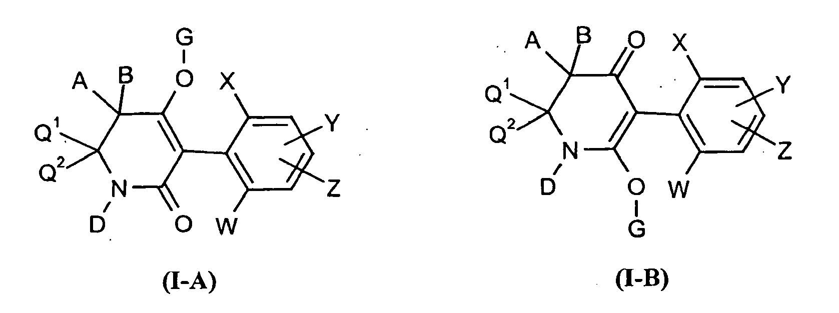 was durch die gestrichelte Linie in der Formel (I) zum Ausdruck gebracht werden soll.

Die Verbindungen der Formeln (I-A) und (I-B) können sowohl als Gemische als auch in Form ihrer reinen Isomeren vorliegen. Gemische der Verbindungen der Formeln (I-A) und (I-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-a) bis (I-g) 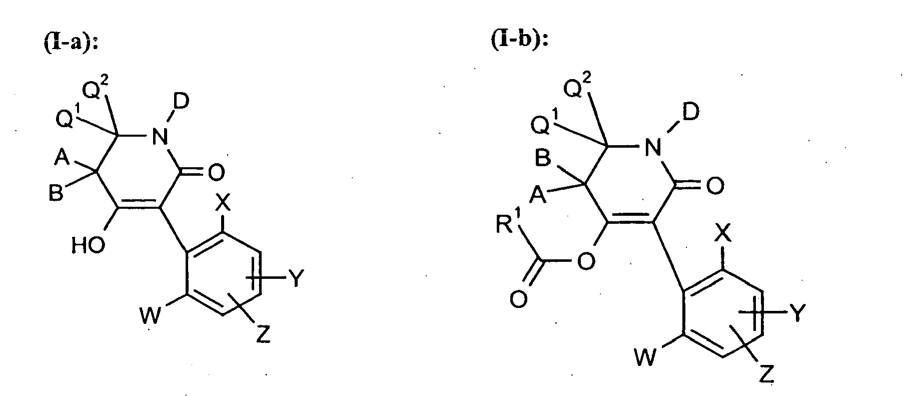 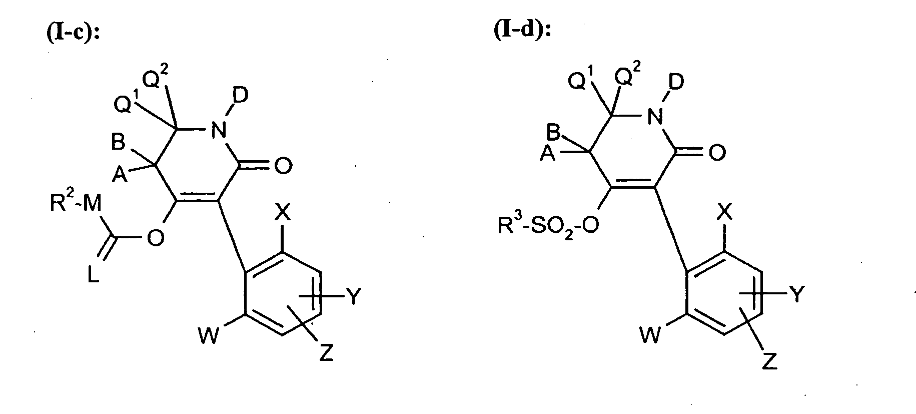 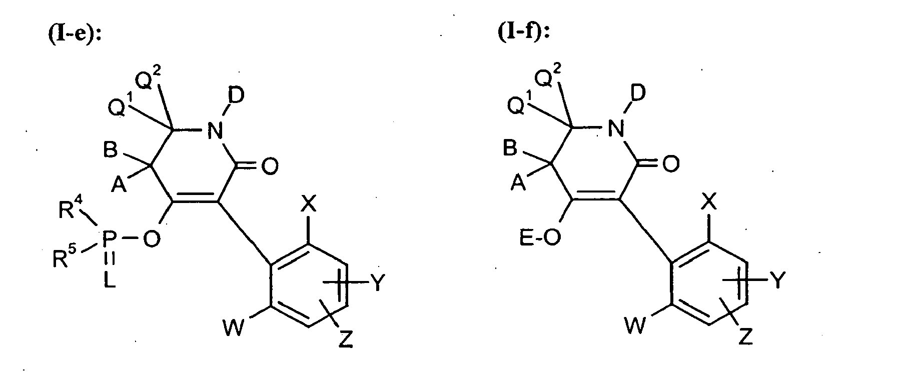 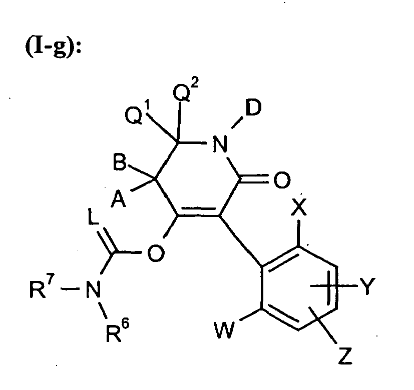 worin
A, B, D, E, L, M, Q¹, Q², W, X, Y, Z, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte Tetrahydropyridin-2,4-dione bzw. deren Enole der Formel (I-a) 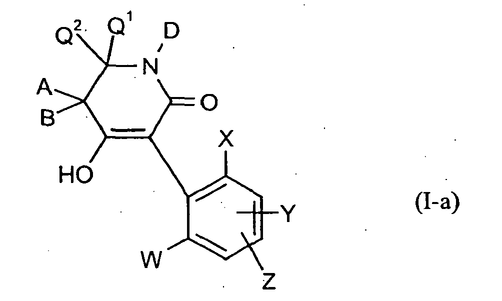 in welcher
   A, B, D, Q¹, Q², W, X, Y und Z die oben angegebenen Bedeutungen haben, wenn man
   N-Acylaminosäureester der Formel (II) 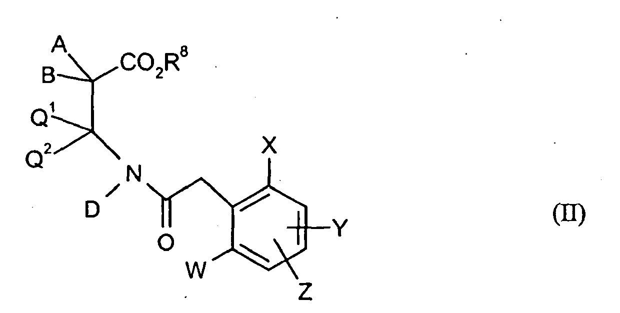 in welcher
   A, B, D, Q1, Q2, W, X, Y und Z die oben angegebenen Bedeutungen haben,
      und
      - R⁸: für Alkyl (bevorzugt C₁-C₆-Alkyl) steht,
      in Gegenwart eines Verdünnungsmittels und in Gegenwart einer Base intramolekular kondensiert.
   Weiterhin wurde gefunden,
(B) dass man Verbindungen der oben gezeigten Formeln (I-a) bis (I-g), in welchen A, B, D, G, Q¹, Q², W, X, Y und Z die oben angegebenen Bedeutungen haben, erhält, wenn man Verbindungen der Formel (I-a') bis (I-g'), 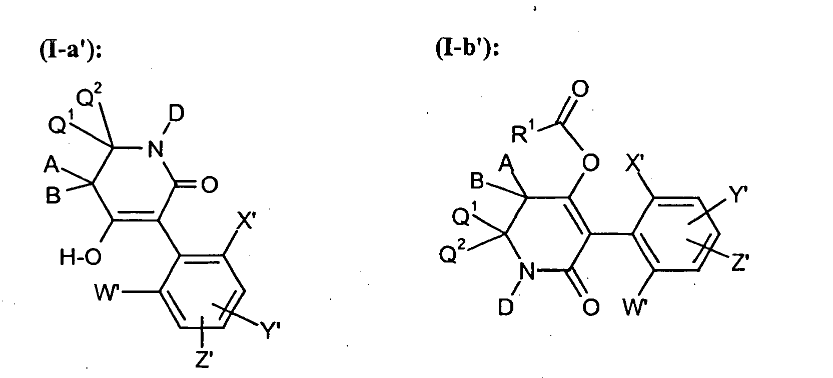 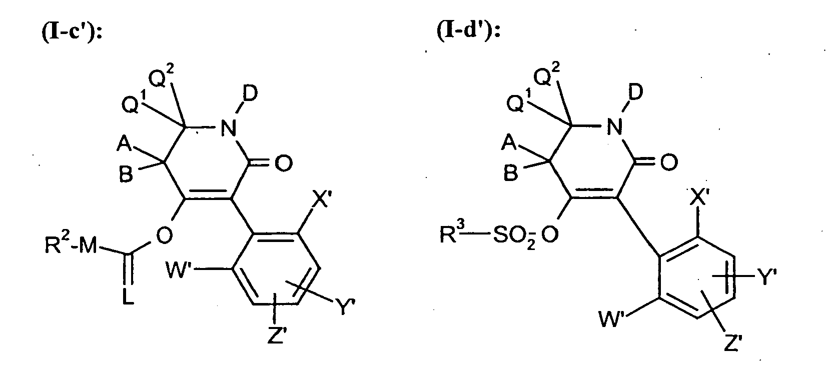 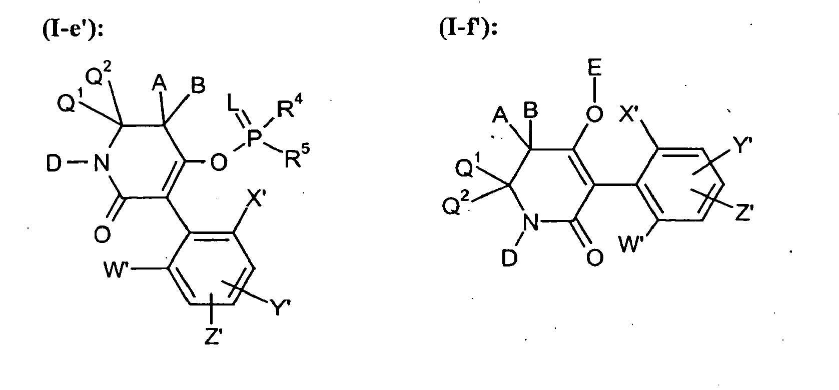 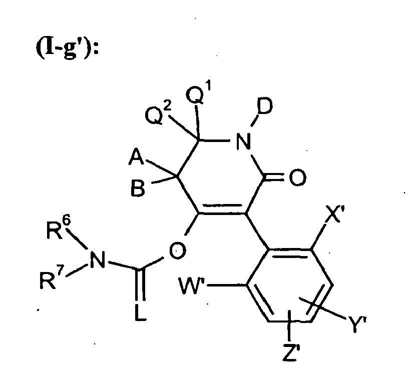 in welchen
   A, B, D, R¹, R², R³, R⁴, R⁵, R⁶, R⁷, E, L, M, Q¹, Q², W', X', Y' und Z' die oben angegebene Bedeutung haben und wobei mindestens einer der Reste
   W', X', Y' für Chlor, Brom oder Jod, bevorzugt für Brom steht,
   und Z' nicht für Brom oder Jod steht,

   α) mit Silylacetylen der Formel (III) 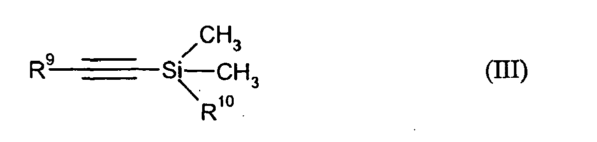 in welcher
      - R⁹: für Wasserstoff und
      - R¹⁰: für C₁-C₄-Alkyl oder Phenyl, besonders für Methyl oder tert.- Butyl steht, zunächst in Gegenwart eines Lösungsmittel, einer Base und eines Katalysators umsetzt, wobei als Katalysator insbesondere Palladium- komplexe in Frage kommen, und anschließend die Silylgruppe ab- spaltet,
      oder
   β) mit Vinylstannanen der Formel (IV) 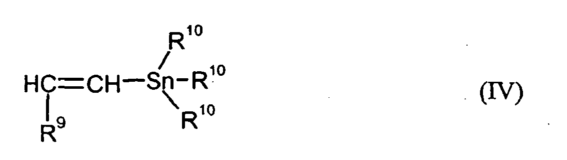 in welcher
      - R⁹: für Wasserstoff, Methyl oder Ethyl und
      - R¹⁰: für C₁-C₄-Alkyl, insbesondere für Butyl steht,
      in Gegenwart eines Lösungsmittels, gegebenenfalls in Gegenwart einer Base und in Gegenwart eines Katalysators umsetzt, wobei als Katalysator insbesondere Palladiumkomplexe in Frage kommen,
      oder
   γ) im speziellen Fall, wenn Y' für Chlor, Brom oder Jod, bevorzugt für Brom steht, mit Boronsäuren der Formel (V) 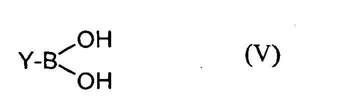 in welcher
      - Y: für gegebenenfalls substituiertes Phenyl oder Hetaryl steht,
      in Gegenwart eines Lösungsmittels, einer Base und eines Katalysators umsetzt, wobei als Katalysator insbesondere Palladiumkomplexe in Frage kommen.
      Außerdem wurde gefunden,
(C) dass man die Verbindungen der oben gezeigten Formel (I-b), in welcher A, B, D, Q¹, Q², R¹, W, X, Y und Z die oben angegebenen Bedeutungen haben, erhält, wenn man Verbindungen der oben gezeigten Formel (I-a), in welchen A, B, D, Q¹, Q², W, X, Y und Z die oben angegebenen Bedeutungen haben, jeweils
   (α) mit Säurehalogeniden der Formel (VI) 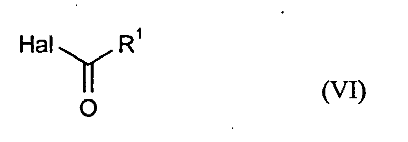 in welcher
      - R¹: die oben angegebene Bedeutung hat und
      - Hal: für Halogen (insbesondere Chlor oder Brom) steht
      oder
   (β) mit Carbonsäureanhydriden der Formel (VII)

      R¹-CO-O-CO-R¹ (VII)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(D) dass man die Verbindungen der oben gezeigten Formel (I-c), in welcher A, B, D, Q¹, Q², R², M, W, X, Y und Z die oben angegebenen Bedeutungen haben und L für Sauerstoff steht, erhält, wenn man Verbindungen der oben gezeigten Formel (I-a), in welcher A, B, D, Q¹, Q², W, X, Y und Z die oben angegebenen Bedeutungen haben, jeweils
   mit Chlorameisensäureestern oder Chlorameisensäurethioestern der Formel (VIII)

   R²-M-CO-Cl (VIII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formel (I-c), in welcher A, B, D, Q¹, Q², R², M, W, X, Y und Z die oben angegebenen Bedeutungen haben und L für Schwefel steht, erhält, wenn man Verbindungen der oben gezeigten Formel (I-a), in welcher A, B, D, Q¹, Q², W, X, Y und Z die oben angegebenen Bedeutungen haben, jeweils
   mit Chlormonothioameisensäureestern oder Chlordithioameisensäureestern der Formel (IX) 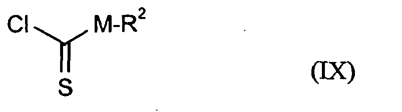 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(F) dass man Verbindungen der oben gezeigten Formel (I-d), in welcher A, B, D, Q¹, Q², R³, W, X, Y und Z die oben angegebenen Bedeutungen haben, erhält, wenn man Verbindungen der oben gezeigten Formel (I-a), in welcher A, B, D, Q¹, Q², W, X, Y und Z die oben angegebenen Bedeutungen haben, jeweils
   mit Sulfonsäurechloriden der Formel (X)

   R³-SO₂-Cl (X)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formel (I-e), in welcher A, B, D, L, Q¹, Q², R⁴, R⁵, W, X, Y und Z die oben angegebenen Bedeutungen haben, erhält, wenn man Verbindungen der oben gezeigten Formel (I-a), in welcher
   A, B, D, Q¹, Q², W, X, Y und Z die oben angegebenen Bedeutungen haben, jeweils
      mit Phosphorverbindungen der Formel (XI) 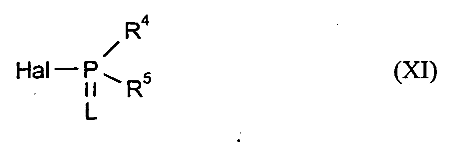 in welcher
      - L, R⁴ und R⁵: die oben angegebenen Bedeutungen haben und
      - Hal: für Halogen (insbesondere Chlor oder Brom) steht,
      gegebenenfalls in Gegenwart eines Verdünnungsmittels und gegebenenfalls in Gegenwart eines Säurebindemittels umsetzt,
(H) dass man Verbindungen der oben gezeigten Formel (I-f), in welcher A, B, D, E, Q¹, Q², W, X, Y und Z die oben angegebenen Bedeutungen haben, erhält, wenn man Verbindungen der Formel (I-a), in welchen A, B, D, Q¹, Q², W, X, Y und Z die oben angegebenen Bedeutungen haben, jeweils
   mit Metallverbindungen oder Aminen der Formeln (XII) oder (XIII)

   Me(OR¹¹)ₜ (XII)

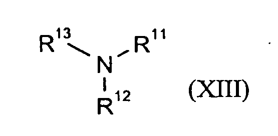

   in welchen
   - Me: für ein ein- oder zweiwertiges Metall (bevorzugt ein Alkali- oder Erd- alkalimetall wie Lithium, Natrium, Kalium, Magnesium oder Calcium),
   - t: für die Zahl 1 oder 2 und
   - R¹¹, R¹², R¹³: unabhängig voneinander für Wasserstoff oder Alkyl (bevor- zugt C₁-C₈-Alkyl) stehen,
   gegebenenfalls in Gegenwart eines Verdünnungsmittels umsetzt,
(I) dass man Verbindungen der oben gezeigten Formel (I-g), in welcher A, B, D, L, Q¹, Q², R⁶, R⁷, W, X, Y und Z die oben angegebenen Bedeutungen haben, erhält, wenn man Verbindungen der oben gezeigten Formel (I-a); in welcher A, B, D, Q¹, Q², W, X, Y und Z die oben angegebenen Bedeutungen haben, jeweils
   (α) mit Isocyanaten oder Isothiocyanaten der Formel (XIV)

      R⁶-N=C=L (XIV)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XV) 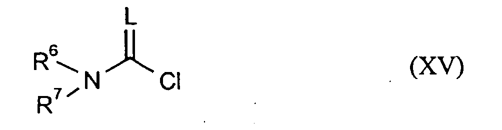 in welcher
      L, R⁶ und R⁷ die oben angegebenen Bedeutungen haben, gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und auch als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:

Insbesondere bevorzugt sind Verbindungen der Formel (I), in denen G für Wasserstoff steht.

Insbesondere bevorzugt sind Verbindungen der Formel (I), in denen D für Wasserstoff steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Verwendet man gemäß Verfahren (A) N-[(2,3,4,6-Tetramethyl)-phenylacetyl]-1-aminomethyl-cyclohexan-carbonsäureethylester als Ausgangsstoff, so kann der Verlauf des erfindungsgemäßen Verfahrens durch folgendes Reaktionsschema wiedergegeben werden:

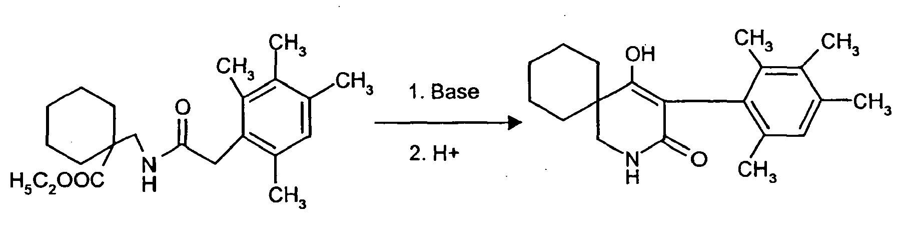

Verwendet man gemäß Verfahren (Bγ) 3-[(2-Chlor-4-brom-6-methyl)-phenyl]-6,6-dimethyl-piperidin-2,4-dion und 4-Chlorphenylboronsäure als Ausgangsprodukte, so kann der Reaktionsverlauf durch folgendes Schema wiedergegeben werden:

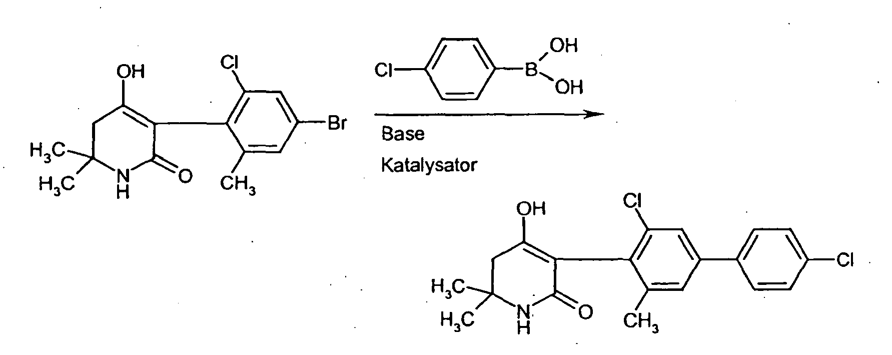

Verwendet man gemäß Verfahren (Cα) 3-[(2,4-Dichlor-6-methyl)-phenyl]-6,6-dimethylpiperidin-2,4-dion und Pivaloylchlorid als Ausgangsstoffe, so kann der Verlauf des erfindungsgemäßen Verfahrens durch folgendes Reaktionsschema wiedergegeben werden:

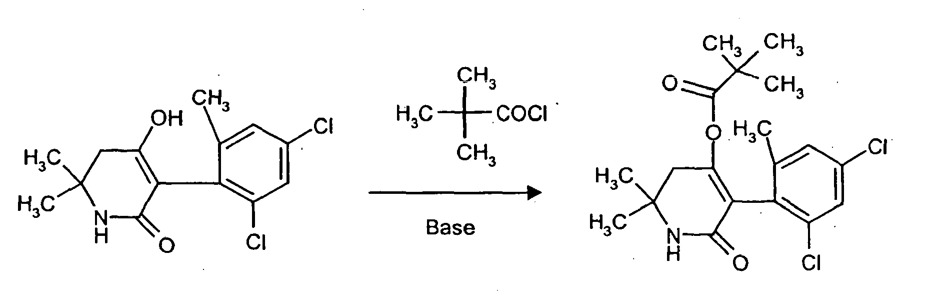

Verwendet man gemäß Verfahren (Cβ) 3-[(4-Brom-2-chlor-6-ethyl)-phenyl]-6,6-dimethyl-piperidin-2,4-dion und Acetanhydrid als Ausgangsverbindungen, so kann der Verlauf des erfindungsgemäßen Verfahrens durch folgendes Reaktionsschema wiedergegeben werden:

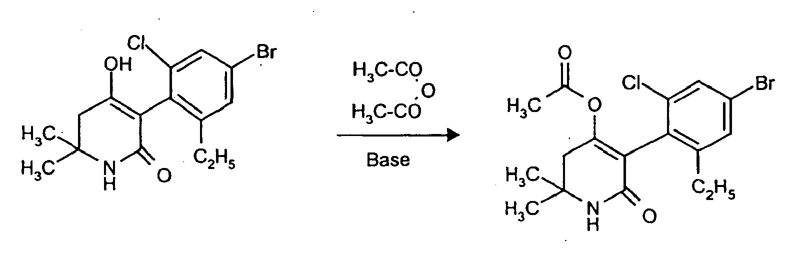

Verwendet man gemäß Verfahren (D) 3-[(2-Chlor-6-ethyl-4-phenyl)-phenyl]-5,5-dimethylpiperidin-2,4-dion und Chlorameisensäureethoxyethylester als Ausgangsverbindungen, so kann der Verlauf des erfindungsgemäßen Verfahrens durch folgendes Reaktionsschema wiedergegeben werden: 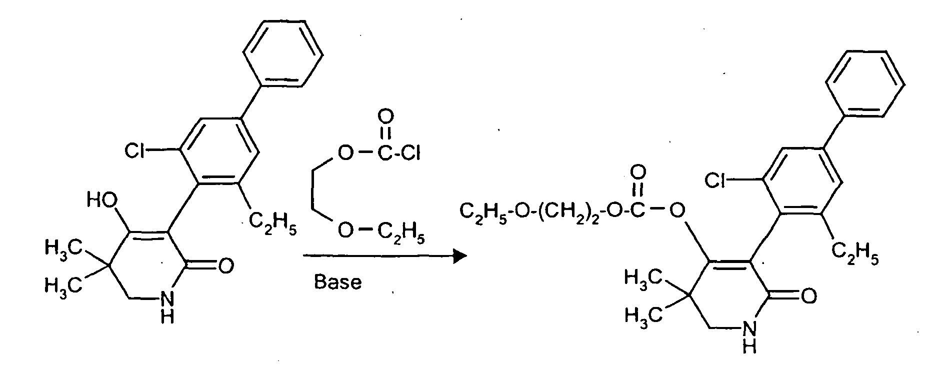 Verwendet man gemäß Verfahren (E), 3-[2,4,6-Trichlor-phenyl]-6,6-dimethyl-piperidin-2,4-dion und Chlormonothioameisensäuremethylester als Ausgangsprodukte, so kann der Reaktionsverlauf folgendermaßen wiedergegeben werden:

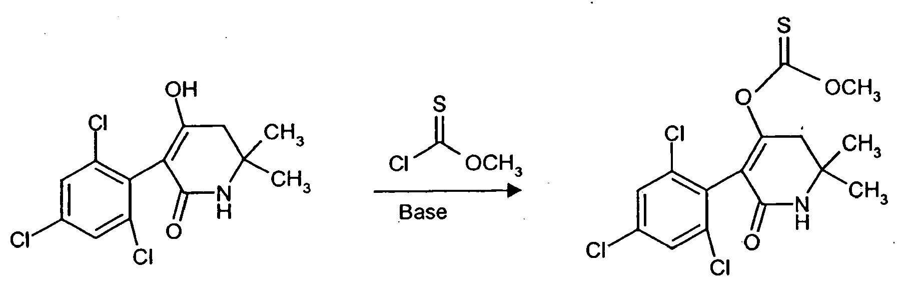

Verwendet man gemäß Verfahren (F) 3-(2,4-Dichlor-6-methyl-phenyl)-6,6-dimethylpiperidin-2,4-dion und Methansulfonsäurechlorid als Ausgangsprodukt, so kann der Reaktionsverlauf durch folgendes Reaktionsschema wiedergegeben werden:

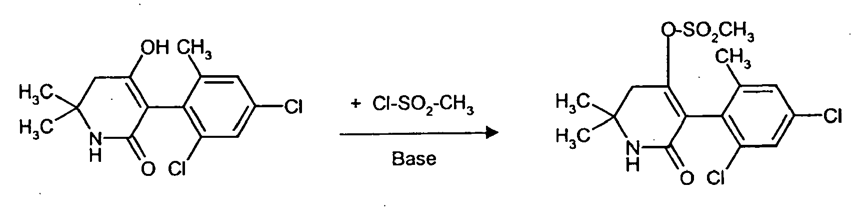

Verwendet man gemäß Verfahren (G) 2-(2-Methyl-5-brom-phenyl]-6,6-dimethylpyridin-2,4-dion und Methanthio-phosphonsäurechlorid-(2,2,2-trifluorethylester) als Ausgangsprodukte, so kann der Reaktionsverlauf durch folgendes Reaktionsschema wiedergegeben werden: 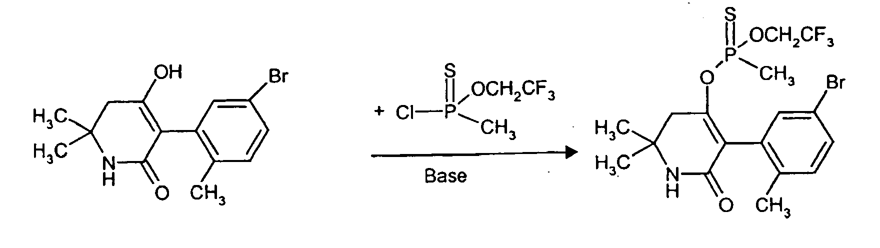 Verwendet man gemäß Verfahren (H) 3-(2,4-Dichlor-phenyl)-6,6-dimethyl-piperidin-2,4-dion und NaOH als Komponenten, so kann der Verlauf des erfindungsgemäßen Verfahrens durch folgendes Reaktionsschema wiedergegeben werden:

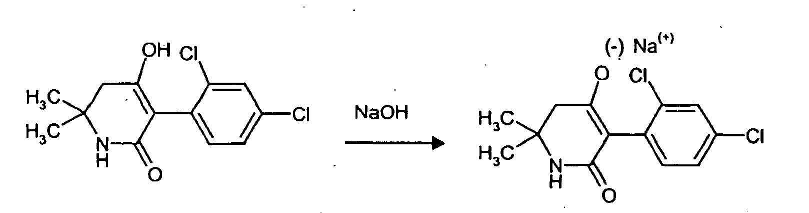

Verwendet man gemäß Verfahren (Iα) 3-(2,4-Dichlor-6-methyl-phenyl)-6,6-dimethyl-piperidin-2,4-dion und Ethylisocyanat als Ausgangsprodukte, so kann der Reaktionsverlauf durch folgendes Reaktionsschema wiedergegeben werden:

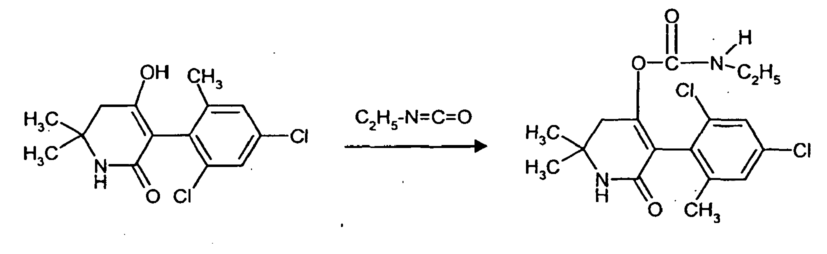

Verwendet man gemäß Verfahren (Iβ) 3-(2-Chlor-4-brom-phenyl)-6,6-dimethylpiperidin-2,4-dion und Dimethylcarbamidsäurechlorid als Ausgangsprodukte, so kann der Reaktionsverlauf durch folgendes Schema wiedergegeben werden: 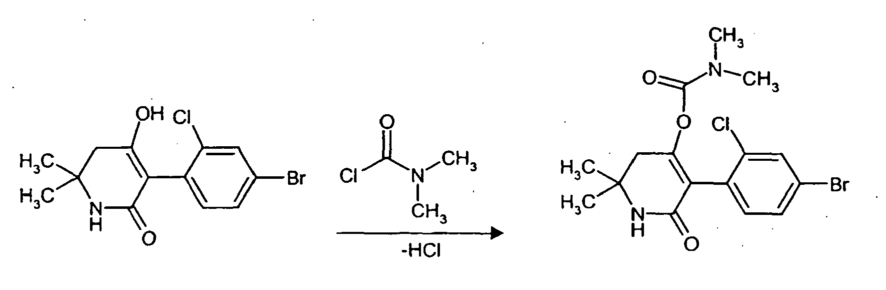 Die beim erfindungsgemäßen Verfahren (A) als Ausgangsstoffe benötigten Verbindungen der Formel (II) 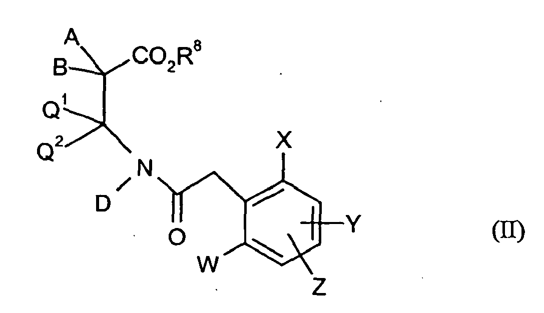 in welcher
A, B, D, Q¹, Q², W, X, Y, Z und R⁸ die oben angegebenen Bedeutungen haben,
sind neu, ausgenommen die Verbindungen 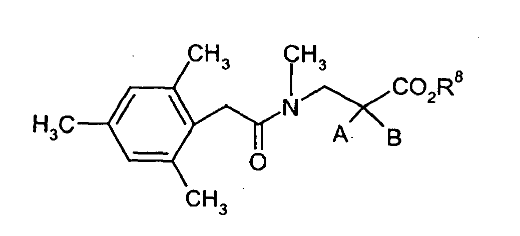
in welcher
- A und B: für Methyl oder
- A und B: für -(CH₂)₅- stehen und
- R⁸: für Alkyl steht.

Man erhält die Acylaminosäureester der Formel (II) beispielsweise, wenn man Aminosäurederivate der Formel (XVI) 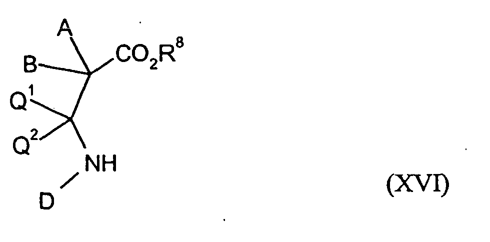 in welcher
- A, B, Q¹, Q², R⁸ und D: die oben angegebenen Bedeutungen haben,
mit substituierten Phenylessigsäurehalogeniden der Formel (XVII) 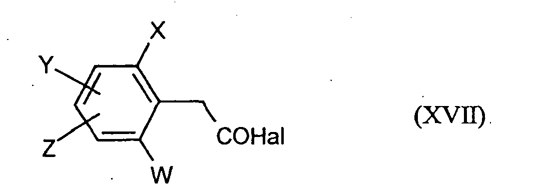 in welcher
- W, X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5,1968)
oder wenn man Acylaminosäuren der Formel (XVIII) 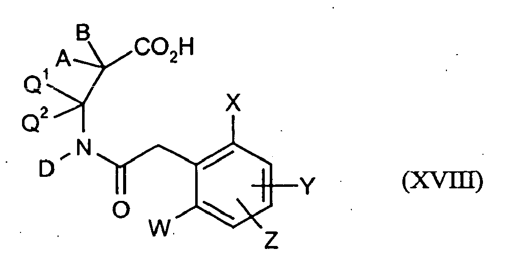 in welcher
- A, B, D, Q¹, Q², W, X, Y und Z: die oben angegebenen Bedeutungen haben,
verestert (Chem. Ind. (London) 1568 (1968)).

Die Verbindungen der Formel (XVIII) 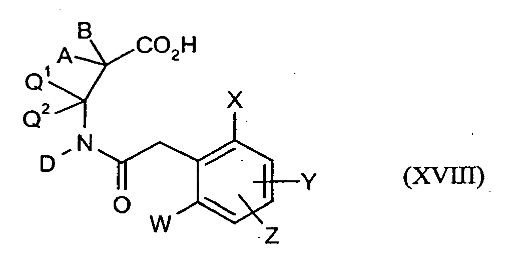 in welcher
- A, B, D, Q¹, Q², W, X, Y und Z: die oben angegebenen Bedeutungen haben,
sind neu.

Man erhält die Verbindungen der Formel (XVIII), wenn man Aminosäuren der Formel (XIX) 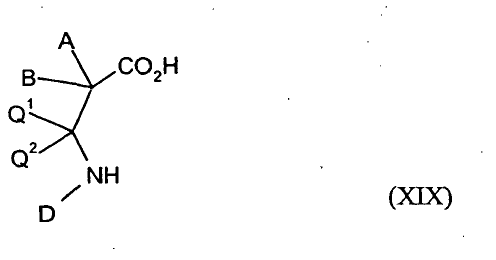 in welcher
- A, B, Q¹, Q² und D: die oben angegebenen Bedeutungen haben,
mit substituierten Phenylessigsäurehalogeniden der Formel (XVII) 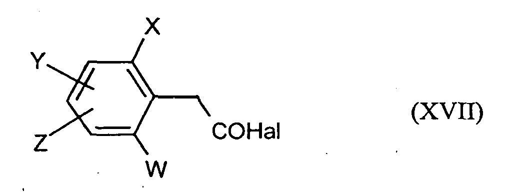 in welcher
- W, X, Y und Z: die oben angegebenen Bedeutungen haben und
- Hal: für Chlor oder Brom steht;
beispielsweise nach Schotten-Baumann acyliert (Organikum, VEB Deutscher Verlag der Wissenschaften, Berlin 1977, S. 505).

Die Verbindungen der Formel (XVII) sind bekannt. Sie lassen sich nach im Prinzip bekannten Verfahren darstellen (s. z.B. H. Henecka, Houben-Weyl, Methoden der Organischen Chemie, Bd. 8, S. 467-469 (1952)).

Man erhält die Verbindungen der Formel (XVII) beispielsweise, indem man substituierte Phenylessigsäuren der Formel (XX) 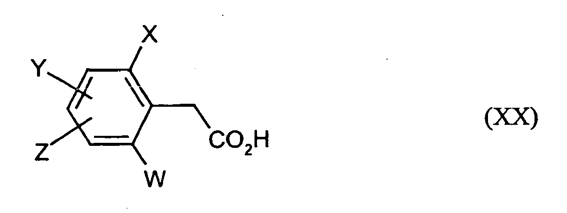 in welcher
- W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XVI) und (XIX) sind teilweise bekannt und/oder lassen sich nach bekannten Verfahren darstellen (siehe z.B. T. Suzuki et al., Synthetic Commun. 28, 701 (1998), R. Graf, Justus Liebigs Ann. Chem. 661, 111 (1963)).

Weiterhin lassen sich die bei dem obigen Verfahren (A) verwendeten Ausgangsstoffe der Formel (II) 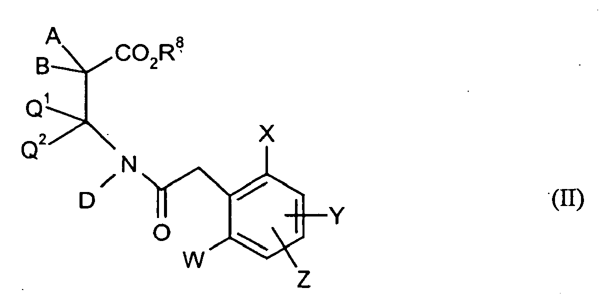 in welcher
- A, B, D, Q¹, Q², W, X, Y, Z und R⁸: die oben angegebenen Bedeutungen haben,
herstellen, wenn man Aminonitrile der Formel (XXI) 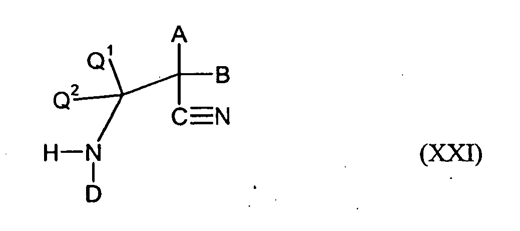 in welcher
- A, B, Q¹, Q² und D: die oben angegebenen Bedeutungen haben,
mit substituierten Phenylessigsäurehalogeniden der Formel (XVII) 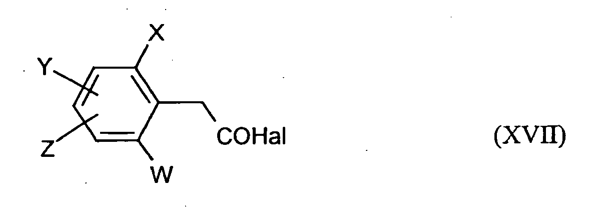 in welcher
- W, X, Y, Z und Hal: die oben angegebenen Bedeutungen haben,
zu Verbindungen der Formel (XXII) 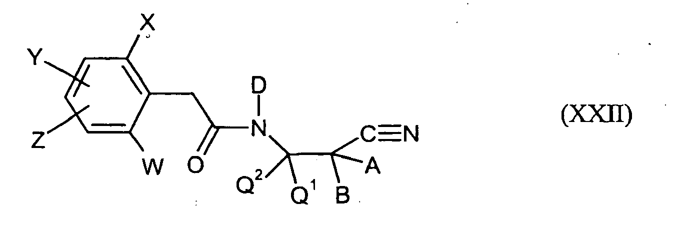 in welcher
- A, B, D, Q¹, Q², W, X, Y und Z: die oben angegebenen Bedeutungen haben,
umsetzt,
und diese anschließend einer sauren Alkoholyse unterwirft.

Die Verbindungen der Formel (XXII) sind ebenfalls neu.

Die Aminonitrile der Formel (XXI) sind teilweise bekannt und/oder lassen sich nach bekannten Verfahren herstellen (T. Suzuki et al., Chem. Pharm. Bull. 46, 1116 (1998)).

Die zur Durchführung des Verfahrens B(α) benötigten Silylacetylene der Formel (III) sind teilweise käuflich oder lassen sich nach allgemein bekannten Verfahren herstellen. Die zur Durchführung des Verfahrens B(β) benötigten Vinylstannane der Formel (IV) sind ebenfalls teilweise käuflich oder lassen sich nach bekannten Verfahren herstellen.

Die zur Durchführung des Verfahrens B(γ) benötigten Boronsäuren der Formel (V) 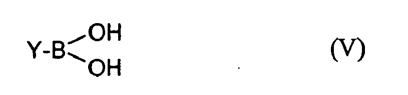 in welcher
- Y: für gegebenenfalls substituiertes Phenyl oder Hetaryl steht,
sind teilweise käuflich oder lassen sich nach allgemein bekannten Verfahren in einfacher Weise herstellen.

Die zur Durchführung der erfindungsgemäßen Verfahren (C), (D), (E), (F), (G), (H) und (I) außerdem als Ausgangsstoffe benötigten Säurehalogenide der Formel (VI) Carbonsäureanhydride der Formel (VII), Chlorameisensäureester oder Chlorameisensäurethioester der Formel (VIII), Chlormonothioameisensäureester oder Chlordithioameisensäureester der Formel (IX), Sulfonsäurechloride der Formel (X), Phosphorverbindungen der Formel (XI) und Metallhydroxide, Metallalkoxide oder Amine der Formel (XII) und (XIII) und Isocyanate der Formel (XIV) und Carbamidsäurechloride der Formel (XV) sind allgemein bekannte Verbindungen der Organischen bzw. Anorganischen Chemie.

Das Verfahren (A) ist dadurch gekennzeichnet, dass man Verbindungen der Formel (II), in welcher A, B, D, Q¹, Q², W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, iso-Propanol, Butanol, iso-Butanol und tert-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -50°C und 200°C, vorzugsweise zwischen -20°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuss (bis zu 3 Mol) zu verwenden.

Zur Durchführung des erfindungsgemäßen Verfahrens B (α) bis B (γ) sind Palladium(0)-Komplexe als Katalysator geeignet. Eingesetzt wird beispielsweise Tetrakis-(triphenylphosphin)palladium. Es eignen sich auch Palladium(II)-Verbindungen wie Bis(triphenylphosphin)palladium(II)chlorid.

Als Säureakzeptoren zur Durchführung des erfindungsgemäßen Verfahrens B (α) und B (γ) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Kaliumfluorid, Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens B (γ) kommen Wasser, organische Lösungsmittel und beliebige Mischungen davon in Betracht. Beispielhaft für die Verfahren B (α) bis B (γ) seien als organische Lösungsmittel genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-tert-butyl-, Methyl-tert-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder iso-Propanol, n-, iso-, sek- oder tert-Butanol, Ethandiol, Propan-1,2-diol, Ethoxyethanol, Methoxyethanol, Diethylenglykolmonomethylether; Diethylenglykolmonoethylether; Wasser.

Die Reaktionstemperatur kann bei dem erfindungsgemäßen Verfahren (B) innerhalb eines größeren Bereiches variiert werden. Im allgemeinen arbeitet man bei Temperaturen zwischen 0°C und +180°C, bevorzugt zwischen 50°C und +150°C.

Bei der Durchführung des Verfahrens B (α) werden Silylacetylene der Formel (III) und Verbindungen der Formel (I-a) bis (I-g) im molaren Verhältnis 1:1 bis 10:1, vorzugsweise 1:1 bis 3:1 eingesetzt. Bei der Durchführung des Verfahrens B(β) werden Vinylstannane der Formel (IV) und Verbindungen der Formel (I-a) bis (I-g) im molaren Verhältnis von 1:1 bis 10:1, bevorzugt 1:1 bis 3:1 eingesetzt.

Bei der Durchführung des erfindungsgemäßen Verfahrens B (γ) werden Boronsäure der Formel (V) und Verbindungen der Formeln (I-a) bis (I-g) im molaren Verhältnis 1:1 1 bis 3:1, vorzugsweise 1:1 1 bis 2:1 eingesetzt.

Vom Katalysator setzt man im allgemeinen 0,005 bis 0,5 mol, vorzugsweise 0,01 mol bis 0,1 mol pro Mol der Verbindungen (I-1-a) bis (I-1-g) ein. Die Base setzt man im allgemeinen als Überschuss ein.

Das Verfahren (C-α) ist dadurch gekennzeichnet, dass man Verbindungen der Formel (I-a) jeweils mit Carbonsäurehalogeniden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformaid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (C-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (C-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-α) werden die Ausgangsstoffe der Formel (I-a) und das Carbonsäurehalogenid der Formel (VI) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C-β) ist dadurch gekennzeichnet, dass man Verbindungen der Formeln (I-a) mit Carbonsäureanhydriden der Formel (VII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (C-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C-β) werden die Ausgangsstoffe der Formel (I-a) und das Carbonsäureanhydrid der Formel (VII) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formel (I-a) jeweils mit Chlorameisensäureestern oder Chlorameisensäurethiolestern der Formel (VIII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestem inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylsulfoxid und Sulfolan.

Die Reaktionstemperaturen können bei der Durchführung des erfindungsgemäßen Verfahrens (D) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formel (I-a) und der entsprechende Chlorameisensäureester bzw. Chlorameisensäurethiolester der Formel (VIII) im allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formel (I-a) jeweils mit Verbindungen der Formel (IX) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Chlormonothioameisensäureester bzw. Chlordithioameisensäureester der Formel (IX) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Nitrile, Ether, Ester, Amide, Sulfone, Sulfoxide, aber auch Halogenalkane.

Vorzugsweise werden Acetonitril, Ethylacetat, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kalium-tert-butylat das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formel (I-a) jeweils mit Sulfonsäurechloriden der Formel (X) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-a) ca. 1 Mol Sulfonsäurechlorid der Formel (X) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ester, Ether, Amide, Nitrile, Sulfone, Sulfoxide oder halogenierte Kohlenwasserstoffe wie Methylenchlorid.

Vorzugsweise werden Acetonitril, Ethylacetat, Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kalium-tert-butylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formel (I-a) jeweils mit Phosphorverbindungen der Formel (XI) gegebenenfalls in Gegenwart eines Verdünnungsmittels und gegebenenfalls in Gegenwart eines Säurebindemittels umsetzt.

Beim Herstellungsverfahren (G) setzt man zum Erhalt von Verbindungen der Formel (I-e) auf 1 Mol der Verbindung (I-a), 1 bis 2, vorzugsweise 1 bis 1,3 Mol der Phosphorverbindung der Formel (XI) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (H) ist dadurch gekennzeichnet, dass man Verbindungen der Formel (I-a) mit Metallhydroxiden bzw. Metallalkoxiden der Formel (XII) oder Aminen der Formel (XIII), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (H) wird im allgemeinen unter Normaldruck durchgeführt.

Die Reaktionstemperaturen liegen im allgemeinen zwischen -20°C und 100°C, vorzugsweise zwischen 0°C und 50°C.

Das erfindungsgemäße Verfahren (I) ist dadurch gekennzeichnet, dass man Verbindungen der Formel (I-a) jeweils mit (I-α) Verbindungen der Formel (XIV) gegebenenfalls in Gegenwart eines Verdünnungsmittels und gegebenenfalls in Gegenwart eines Katalysators oder (I-β) mit Verbindungen der Formel (XV) gegebenenfalls in Gegenwart eines Verdünnungsmittels und gegebenenfalls in Gegenwart eines Säurebindemittels umsetzt.

Bei Herstellungsverfahren (I-α) setzt man pro Mol Ausgangsverbindung der Formel (I-a) ca. 1 Mol Isocyanat der Formel (XIV) bei 0 bis 100°C, vorzugsweise bei 20 bis 50°C um.

Als gegebenenfalls zugesetzte Verdünnungsmittel kommen alle inerten organischen Lösungsmittel in Frage, wie Nitrile, Ester, Ether, Amide, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (I-β) setzt man pro Mol Ausgangsverbindung der Formel (I-a) ca. 1 Mol Carbamidsäurechlorid der Formel (XV) bei -20 bis 150°C, vorzugsweise bei 0 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe.

Vorzugsweise werden Acetonitril, Ethylenacetat, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kalium-tert-butylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z:B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Onithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin, Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2, 5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol;
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholin-hydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinarnin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid, N-[2,2,2-Trichlor-1-[(chloracetyl)-amino]-ethyl]-benzamid,
N-[3-Chlor-4-5-bis-(2-propinyloxy)-phenyl]-N-methoxy-methanimidamid,
N-Formyl-N-hydroxy-DL-alanin -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide

Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
Deltamethrin, Demeton M, Demetön S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren
Halofenozide, HCH, Heptenophos, Hexaflumuron, Hexythiazox, Hydroprene, Imidacloprid, Isazofos, Isofenphos, Isoxathion, Ivermectin,
Kernpolyederviren
Lambda-cyhalothrin, Lufenuron
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
Naled, Nitenpyram, Nithiazine, Novaluron
Omethoat, Oxamyl, Oxydemethon M
Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
Quinalphos,
Ribavirin
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecanii
YI 5302
Zeta-cypermethrin, Zolaprofos
(1R-cis)-[5-(Phenylmethyl)-3-furanyl]-methyl-3-[(dihydro-2-oxo-3(2H)-furanyliden)-methyl]-2,2-dimethylcyclopropancarboxylat
(3-Phenoxyphenyl)-methyl-2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
2-(2-Chlor-6-fluorphenyl)-4-[4-(1,1-dimethylethyl)phenyl]-4,5-dihydro-oxazol 2-(Acetyloxy)-3-dodecyl-1,4-naphthalindion
2-Chlor-N-[[[4-(1-phenylethoxy)-phenyl]-amino]-carbonyl]-benzamid
2-Chlor-N-[[[4-(2,2-dichlor-1,1-difluorethoxy)-phenyl]-amino]-carbonyl]-benzamid 3-Methylphenyl-propylcarbamat
4-[4-(4-Ethoxyphenyl)-4-methylpentyl]-1-fluor-2-phenoxy-benzol
4-Chlor-2-(1,1-dimethylethyl)-5-[[2-(2,6-dimethyl-4-phenoxyphenoxy)ethyl]thio]-3(2H)-pyridazinon
4-Chlor-2-(2-chlor-2-methylpropyl)-5-[(6-iod-3-pyridinyl)methoxy]-3 (2H)-pyridazinon
4-Chlor-5-[(6-chlor-3-pyridinyl)methoxy]-2-(3,4-dichlorphenyl)-3(2H)-pyridazinon Bacillus thuringiensis strain EG-2348
Benzoesäure [2-benzoyl-1-(1,1-dimethylethyl)-hydrazid
Butansäure 2,2-dimethyl-3-(2,4-dichlorphenyl)-2-oxo-1-oxaspiro[4.5]dec-3-en-4-yl-ester
[3-[(6-Chlor-3-pyridinyl)methyl]-2-thiazolidinyliden]-cyanamid
Dihydro-2-(nitromethylen)-2H-1,3-thiazine-3(4H)-carboxaldehyd
Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
N-(4-Chlorphenyl)-3-[4-(difluormethoxy)phenyl]-4,5-dihydro-4-phenyl-1H-pyrazol-1-carboxamid
N-[(2-Chlor-5-thiazolyl)methyl]-N'-methyl-N"-nitro-guanidin
N-Methyl-N'-(1-methyl-2-propenyl)-1,2-hydrazindicarbothioamid
N-Methyl-N'-2-prop enyl-1,2-hydrazindicarbothioamid
O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstofikkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec., Tryptodendron spec., Apate monachus, Bostrychus capucins, Heterobostrychus brunneus, Sinoxylon spec., Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001. bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel-(gemisch) oder ein Weichmacher(gemisch) ersetzt werden. Diese Zusätze sollen einer Verflüchtigung der Wirkstoffe sowie einer Kristallisation bzw. einem Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyfenozide und Triflumuron,

sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, daß die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-tert-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb;
oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposeelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp.; Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Das können Sorten, Bio- oder Genotypen, sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften. ("Traits") werden auch besonders hervorgehoben die erhöhte Abwehr von Pflanzen gegen Pilze, Bakerien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der Formel (I) behandelt werden. Die bei den Wirkstoffen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

### Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, BAS-662H, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Brombutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele

### Beispiel I-a-1

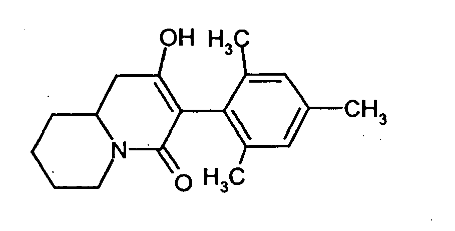

3,9 g (0,13 mol) Natriumhydrid werden in 70 ml absolutem Toluol unter Rückfluß erhitzt und 33,1 g der Verbindung gemäß Beispiel II-1 in 100 ml absolutem Toluol zugetropft. Die Reaktion wird mittels Dünnschichtchromatographie verfolgt. Unter Eiskühlung tropft man Ethanol hinzu bis kein Wasserstoff mehr entweicht, dampft das Lösungsmittel ein und gibt Wasser hinzu. Nach Abtrennung der Toluolphase wird bei 0-20°C die wässrige Phase mit konzentrierter Salzsäure angesäuert, der Niederschlag abfiltriert und getrocknet.

Die Reinigung des Produktes erfolgt säulenchromatographisch an Kieselgel (Dichlormethan/Essigsäureethylester 3:1).
Ausbeute: 8,41 g (30 % d. Th.), Fp. 127°C.

### Beispiel I-a-2

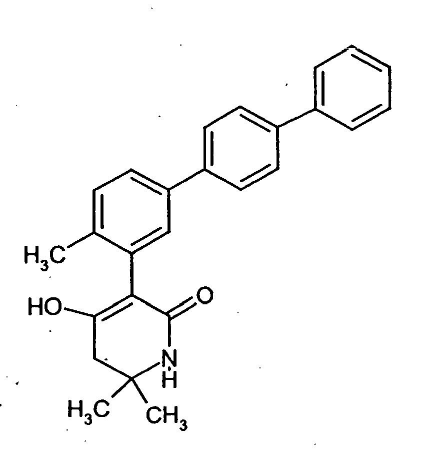

3 mmol der Verbindung gemäß Beispiel I-a-4 werden in 8 ml DME (Dinethoxyethan) bei Raumtemperatur vorgelegt und 6,9 ml einer 1M NaCO₃-Lösung hinzugegeben. 4,5 mmol Biphenylboronsäure wird hinzugegeben, anschließend gibt man 0,15 mmol Pd(PPh₃)₂ Cl₂ zu und rührt 1 h bei Raumtemperatur, anschließend 16 h bei 85°C.

Die Reinigung erfolgte an Kieselgel mit Cyclohexan/Essigester (Gradient 100:1 bis 1:1) als Laufmittel.
Ausbeute: 0,302 g (26,3 % d.Th.), Öl.

**Tabelle 1**

| | | | | | | | | | | |
|---|---|---|---|---|---|---|---|---|---|---|
| In Analogie zu den Beispielen (I-a-1) und (I-a-2) und gemäß den allgemeinen Angaben zur Herstellung wurden folgende Verbindungen der Formel (I-a) erhalten | | | | | | | | | | |
| 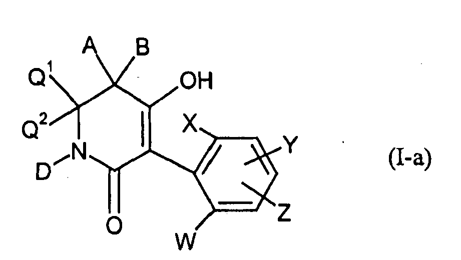 | | | | | | | | | | |

| Bsp.-Nr. | W | X | Y | Z | A | B | D | Q¹ | Q² | Fp°C |
|---|---|---|---|---|---|---|---|---|---|---|
| I-a-3 | CH₃ | Cl | 4-Cl | H | H | H | H | CH₃ | CH₃ | 223 |
| I-a-4 | H | CH₃ | 5-Br | H | H | H | H | CH₃ | CH₃ | 242 |
| I-a-5 | H | CH₃ | 5-(4-Cl-C₆H₅) | H | H | H | H | CH₃ | CH₃ | 186 |
| I-a-6 | H | CH₃ | 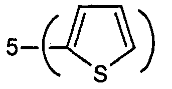 | H | H | H | H | CH₃ | CH₃ | Öl |
| I-a-7 | H | CH₃ | 5-(3,5-Cl₂-C₆H₃) | H | H | H | H | CH₃ | CH₃ | Öl |
| I-a-8 | H | CH₃ | 5-(3-Cl-C₆H₄) | H | H | H | H | CH₃ | CH₃ | Öl |
| I-a-9 | H | CH₃ | 5-(3,5-(CF₃)₂-C₆H₃) | H | H | H | H | CH₃ | CH₃ | Öl |
| I-a-10 | CH₃ | Cl | 4-Br | H | H | H | H | CH₃ | CH₃ | >245 |
| I-a-11 | C₂H₅ | Cl | 4-Br | H | H | H | H | CH₃ | CH₃ | 188 |
| I-a-21 | Cl | Cl | H | H | H | H | -(CH₂)₄- | | H | 206 |
| I-a-22 | H | Cl | 4-Cl | H | H | H | -(CH₂)₄- | | H | 182 |
| I-a-24 | H | CH₃ | 5-(4-Cl-C₆H₄) | H | H | H | -(CH₂)₄- | | H | 196 |
| I-a-25 | H | CH₃ | 5-(4-Cl-C₆H₄) | H | H | H | CH₃ | CH₃ | CH₃ | 196 |
| I-a-26 | CH₃ | Cl | 4-Cl | H | H | H | 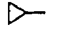 | CH₃ | CH₃ | 191 |
| I-a-27 | C₂H₅ | Cl | 4-Br | H | H | H | CH₃ | CH₃ | CH₃ | |
| I-a-30 | CH₃ | CH₃ | 4-(4-Cl-C₆H₄) | H | H | H | -(CH₂)₄ | | H | 315 |
| I-a-31 | CH₃ | Cl | 4-Cl | H | H | H | H | -(CH₂)₂-O-(CH₂)₂- | | 311 |
| I-a-32 | H | CH₃ | 5-(4-Cl-C₆H₄) | H | H | H | H | -(CH₂)₂-O-(CH₂)₂- | | 154 |
| I-a-33 | H | CH₃ | 5-(4-Cl-C₆H₄) | H | H | H | H | -(CH₂)₂-CHOCH₃-(CH₂)₂- | | |
| I-a-34 | CH₃ | Cl | 4-Cl | H | H | H | H | -(CH₂)₂-CHOCH₃-(CH₂)₂- | | |

### Beispiel I-b-1

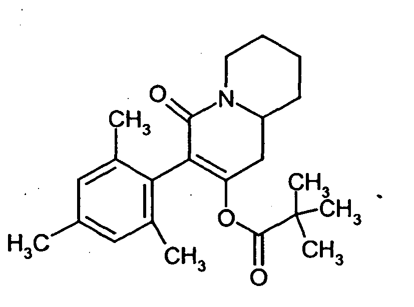

Zu 4,95 g der Verbindung gemäß Beispiel I-a-1 in 70 ml Methyl-tert-butylether gibt man 1,4 ml absolutes Pyridin und 2,94 ml Ethyl-diisopropylamin bei 0-10°C. Anschießend gibt man 2,2 ml Pivaloylchlorid mit 5 ml Methyl-tert-butylether hinzu und rührt bei Raumtemperatur. Man saugt ab, wäscht mit Methyl-tert-butylether und dampft die Lösung ein. Das Reaktionsgemisch wird säulenchromatographisch gereinigt (Cyclohexan/Essigsäureethylester 1:1).
Ausbeute: 3,42 g (55 % d.Th.), Öl

**Tabelle 2**

| | | | | | | | | | | | |
|---|---|---|---|---|---|---|---|---|---|---|---|
| In Analogie zu Beispiel I-b-1 und gemäß den allgemeinen Angaben zur Herstellung erhält man folgende Verbindungen der Formel (I-b) | | | | | | | | | | | |
| 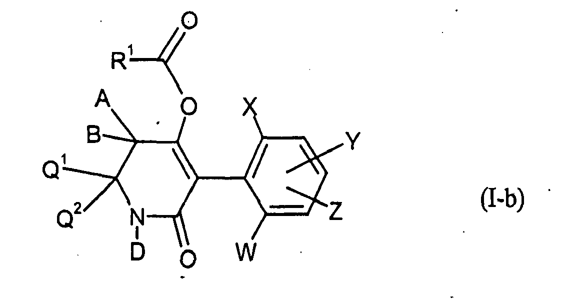 | | | | | | | | | | | |

| Bsp.-Nr. | W. | X | Y | Z | A | B | D | Q¹ | Q² | R¹ | Fp°C |
|---|---|---|---|---|---|---|---|---|---|---|---|
| I-b-2 | Cl | Cl | H | H | H | H | -(CH₂)₄- | | H | CH₃ | 124 |
| I-b-3 | Cl | Cl | H | H | H | H | -(CH₂)₄- | | H | t-C₄H₉ | 130 |
| I-b-5 | H | CH₃ | 5-(4-Cl-C₆H₄) | H | H | H | H | CH₃ | CH₃ | i-C₃H₇ | 167 |
| I-b-6 | H | CH₃ | 5-(4-Cl-C₆H₄) | H | H | H | H | CH₃ | CH₃ | 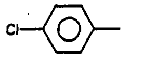 | 199 |
| I-b-7 | H | CH₃ | 5-(4-Cl-C₆H₄) | H | H | H | H | CH₃ | CH₃ | 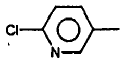 | 234 |
| I-b-8 | H | CH₃ | 5-(4-Cl-C₆H₄) | H | H | H | H | CH₃ | CH₃ | 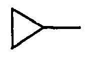 | 178 |

### Beispiel I-c-1

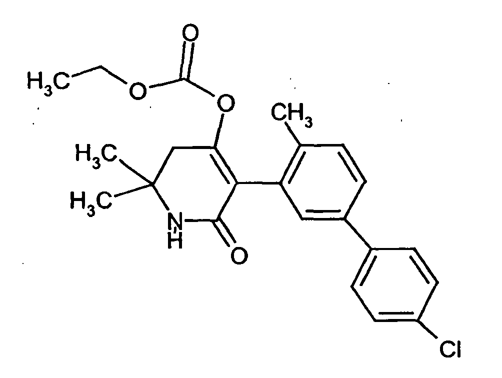

Zu 1,3 g der Verbindung gemäß Beispiel I-a-5 in 30 ml absolutem Dichlormethan gibt man 0,42 ml Triethylamin bei 10-20°C, und anschließend fügt man 0,3 ml Chlorameisensäureethylester in 2 ml absolutem Dichlormethan hinzu und rührt bei Raumtemperatur. Das Reaktionsgemisch wird zweimal mit 10 ml 0,5 N NaOH gewaschen, die Lösung über Magnesiumsulfat getrocknet und einrotiert. Die Reinigung erfolgt säulenchromatographisch an Kieselgel (Dichlormethan/Essigsäureethylester, 3/1).
Ausbeute: 1,1 g (88 % d.Th.) Fp. 195°C.

In Analogie zu Beispiel I-c-1 und gemäß den allgemeinen Angaben zur Herstellung wurden folgende Verbindungen der Formel (I-c) erhalten:

| | | | | | | | | | | | | | |
|---|---|---|---|---|---|---|---|---|---|---|---|---|---|
| 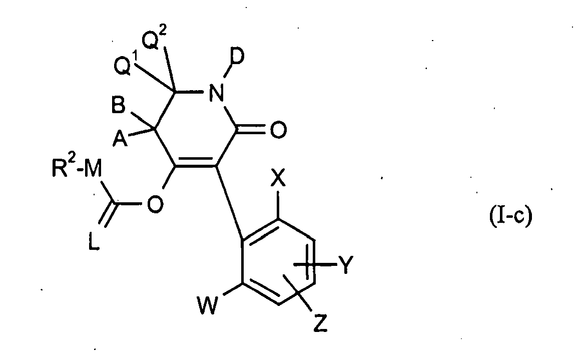 | | | | | | | | | | | | | |

| Bsp.-Nr. | w | X | Y | Z | A | B | D | Q¹ | Q² | L | M | R² | Fp°C |
|---|---|---|---|---|---|---|---|---|---|---|---|---|---|
| I-c-4 | H | CH₃ | 4-(4-Cl-C₆H₅) | H | H | H | H | CH₃ | CH₃ | O | O | 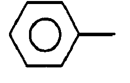 | 180 |
| I-c-5 | H | CH₃ | 5-(4-Cl-C₆H₄) | H | H | H | H | CH₃ | CH₃ | O | O | 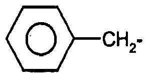 | 216 |
| I-c-6 | H | CH₃ | 5-(4-Cl-C₆H₄) | H | H | H | H | CH₃ | CH₃ | O | S | 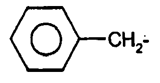 | 165-167 |

### Beispiel I-d-1

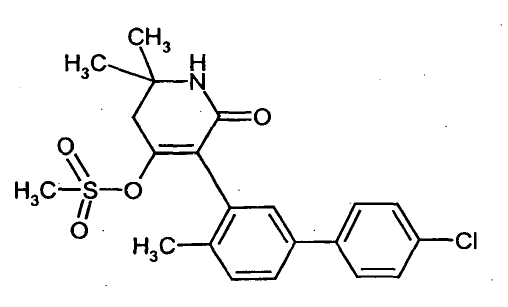

1,02 g (3 mmol) der Verbindung gemäß Herstellungsbeispiel I-a-5 werden in 10 ml absolutem Dichlormethan mit 0,54 ml Triethylamin versetzt. 0,26 ml (3,3 mmol) Methansulfonsäurechlorid werden in etwas Dichlormethan gelöst und unter Eiskühlung zugetropft. Es wird 2 h bei Raumtemperatur gerührt. Die Reaktionslösung wird mit 10 %iger Zitronensäure gewaschen und mit Dichlormethan extrahiert. Die organische Phase wird mit 1 N NaOH-Lösung gewaschen und getrocknet. Ausbeute: 1,05 g (83 % d.Th.). Fp. 198-200°C.

### Beispiel I-A-g-1

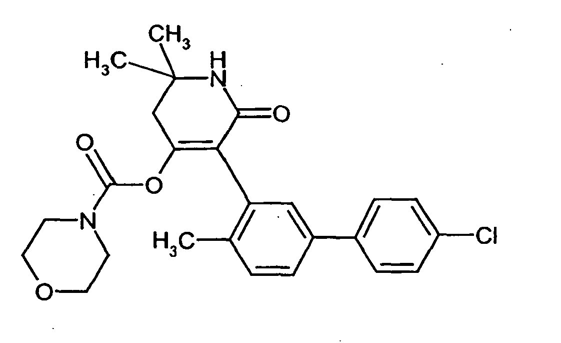

1,02 g (3,0 mmol) der Verbindung gemäß Herstellungsbeispiel I-a-5 und 0,54 ml (3,90 mmol) Triethylamin werden in 10 ml absolutem Essigsäureethylester gelöst und unter Rückfluss erhitzt. Dazu werden 0,47 g (3,15 mmol) Morpholin-N-carbonsäurechlorid in 2 ml absolutem Essigsäureethylester gegeben. Es wird 2 h unter Rückfluss erhitzt.

Das Lösungsmittel wird abdestilliert und der Rückstand in 50 ml Dichlormethan aufgenommen. Es wird 2 mal mit 30 ml ½ konzentrierter NaCl-Lösung gewaschen und 2 mal mit 30 ml 0,5 N NaOH-Lösung extrahiert. Die organische Phase wird getrocknet und eingeengt. Man reinigt säulenchromatographisch über Kieselgel (Cyclohexan/Essigsäureethylester, 5:1 → 1:1). Ausbeute 0,200 g (15 % d.Th.), Fp. 221-223°C.

### Beispiel I-B-g-1

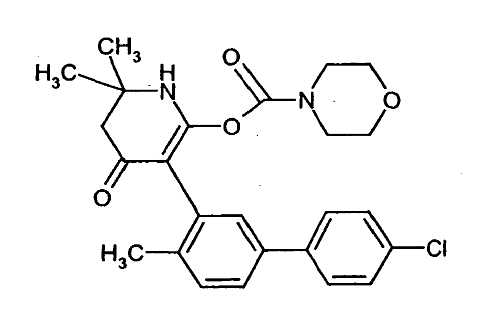

1,02 g (3,0 mmol) der Verbindung gemäß Herstellungsbeispiel I-a-5 und 0,54 ml (3,90 mmol) Triethylamin werden in 10 ml absolutem Essigsäureethylester gelöst und unter Rückfluss erhitzt. Dazu werden 0,47 g (3,15 mmol) Morpholin-N-carbonsäure in 2 ml absolutem Essigsäureethylester gegeben. Es wird 2 h unter Rückfluss erhitzt.

Das Lösungsmittel wird abdestilliert und der Rückstand in 50 ml Dichlormethan aufgenommen. Es wird 2 mal mit 30 ml ½ konzentrierter NaCl-Lösung gewaschen und 2 mal mit 30 ml 0,5 N NaOH-Lösung extrahiert. Die organische Phase wird getrocknet und eingeengt. Man reinigt säulenchromatographisch über Kieselgel (Cyclohexan/Essigsäureethylester, 5:1 → 1:1). Ausbeute 0,480 g (35 % d.Th.), Fp. 95-97°C.

### Beispiel II-1

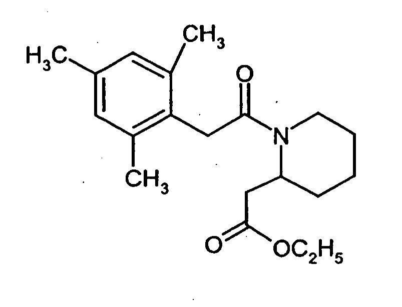

Zu 17,1 g 2-Piperidinyl-essigsäure-ethylester in 130 ml absolutem Tetrahydrofuran gibt man bei 0-10°C 14 ml Triethylamin. Anschließend werden bei Raumtemperatur 19,7 g Mesitylenessigsäurechlorid in 20 ml absolutem Tetrahydrofuran zugegeben.

Die Reaktionslösung wird in 0,5 1 Eiswasser gegeben und mit 100 ml 1N HCl angesäuert. Die Lösung wird mit Dichlormethan extrahiert, getrocknet und das Lösungsmittel eingedampft.
Ausbeute: 33,4 g (100 % d.Th.)

**Tabelle 3**

| | | | | | | | | | | | |
|---|---|---|---|---|---|---|---|---|---|---|---|
| In Analogie zu Beispiel II-1 und gemäß den allgemeinen Angaben zur Herstellung erhält man folgende Verbindungen der Formel (II) | | | | | | | | | | | |
| 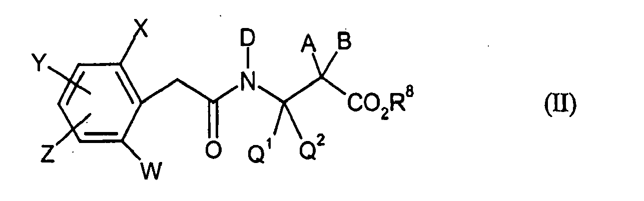 | | | | | | | | | | | |

| Bsp.-Nr. | W | X | Y | Z | A | B | D | Q¹ | Q² | R⁸ | Fp°C |
|---|---|---|---|---|---|---|---|---|---|---|---|
| II-2 | Cl | Cl | H | H | H | H | -(CH₂)₄- | | H | C₂H₅ | 89 |
| II-3 | H | CH₃ | 5-Br | H | H | H | H | CH₃ | CH₃ | C₂H₅ | 101 |
| II-4 | CH₃ | Cl | 4-Cl | H | H | H | H | CH₃ | CH₃ | C₂H₅ | 87 |
| II-5 | H | CH₃ | 5-(4-Cl-C₆H₄) | H | H | H | H | CH₃ | CH₃ | C₂H₅ | 94 |
| II-6 | CH₃ | Cl | 4-Br | H | H | H | H | CH₃ | CH₃ | C₂H₅ | 118 |
| II-7 | C₂H₅ | Cl | 4-Br | H | H | H | H | CH₃ | CH₃ | C₂H₅ | 113 |
| II-18 | H | Cl | 4-Cl | H | H | H | -(CH₂)₄- | | H | C₂H₅ | Öl |
| II-19 | H | CH₃ | 5-(4-Cl-C₆H₄) | H | H | H | -(CH₂)₄- | | H | C₂H₅ | Öl |
| II-20 | CH₃ | CH₃ | 4-(4-Cl-C₆H₄) | H | H | H | -(CH₂)₄- | | H | C₂H₅ | 115 |
| II-21 | CH₃ | Cl | 4-Cl | H | H | H | 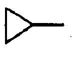 | CH₃ | CH₃ | C₂H₅ | Öl |
| II-22 | H | CH₃ | 5-(4-Cl-C₆H₄) | H | H | H | 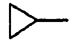 | CH₃ | CH₃ | C₂H₅ | Öl |
| II-23 | H | CH₃ | 5-(4-Cl-C₆H₄) | H | H | H | CH₃ | CH₃ | CH₃ | C₂H₅ | Öl |
| II-24 | Cl | C₂H₅ | 4-Br | H | H | H | CH₃ | CH₃ | CH₃ | C₂H₅ | Öl |
| II-25 | CH₃ | CH₃ | 6-CH₃ | H | H | H | CH₃ | CH₃ | CH₃ | C₂H₅ | Öl |
| II-28 | CH₃ | Cl | 4-Cl | H | H | H | H | -(CH₂)₂-O-(CH₂)₂- | | C₂H₅ | 117 |
| II-29 | CH₃ | CH₃ | 4-Cl | H | H | H | H | -(CH₂)₂-O-(CH₂)₂- | | C₂H₅ | 117 |
| II-30 | H | CH₃ | 5-(4-Cl-C₆H₄) | H | H | H | H | -(CH₂)₂-O-(CH₂)₂- | | C₂H₅ | 118 |
| II-31 | CH₃ | Cl | 4-(4-Cl-C₆H₅) | H | H | H | H | CH₃ | CH₃ | C₂H₅ | 99 |
| II-32 | C₂H₅ | Cl | 4-(4-Cl-C₆H₅) | H | H | H | H | CH₃ | CH₃ | C₂H₅ | Öl |
| II-33 | CH₃ | Cl | 4-Cl | H | H | H | H | -(CH₂)₂-CHOCH₃-(CH₂)₂- | | C₂H₅ | 113 |
| II-35 | H | CH₃ | 5-(4-Cl-C₆H₄) | H | H | H | H | -(CH₂)₂-CHOCH₃-(CH₂)₂- | | C₂H₅ | 124 |
| II-36 | C₂H₅ | Cl | 5-Br | H | H | H | H | (CH₂)₂- CHOCH₃- (CH₂)₂- | | C₂H₅ | 115 |

### Anwendunssbeispiele

### Beispiel A

### Meloidogyne-Test

- Lösungsmittel:: 30 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigte die Verbindung des Herstellungsbeispiels I-a-4 bei einer beispielhaften Wirkstoffkonzentration von 20 ppm eine Abtötung von 95 % nach 14 Tagen.

### Beispiel B

### Tetranychus-Test (OP-resistent/Tauchbehandlung)

- Lösungsmittel:: 30 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. Verbindungen der Herstellungsbeispiele I-a-5 und I-c-1 bei einer beispielhaften Wirkstoffkonzentration von 100 ppm eine Abtötung von 99 % (I-a-5) bzw. 95 % (I-c-1) nach 7 Tagen.

### Beispiel C

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffinengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Verrichtung |

### Beispiel D

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt, so dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0%= | keine Wirkung (wie unbehandelte Kontrolle) |
| 100%= | totale Vernichtung. |

| post-emergence/ Gewächshaus | g/ha | Zuckerrüben | | Alopecurus | Echinochloa | Setaria | Amaranthus |
|---|---|---|---|---|---|---|---|
| Bsp. I-a-10 | 2000 | 0 | | 70 | 80 | 80 | 70 |

| pre-emergence/ Gewächshaus | g/ha | Weizen | Soja | Digitaria | Echinochloa | Lolium | Setaria |
|---|---|---|---|---|---|---|---|
| Bsp. I-a-10 | 250 | 0 | 0 | 90 | 80 | 100 | 95 |

| post-emergence/ Gewächshaus | g/ha | Zuckerrüben | Alopecurus | Avena fatua | Echinochloa | Setaria |
|---|---|---|---|---|---|---|
| Bsp. I-a-11 | 2000 | 90 | 95 | 100 | 100 | 100 |

| pre-emergence/ Gewächshaus | g/ha | Alopecurus | Avena fatua | Echinochloa | Setaria |
|---|---|---|---|---|---|
| Bsp. I-a-11 | 2000 | 100 | 100 | 100 | 100 |

| post-emergence/ Gewächshaus | g/ha | Zuckerrüben | Echinochloa | Setaria | Amaranthus |
|---|---|---|---|---|---|
| Bsp. I-c-1 | 250 | 0 | 70 | 80 | 80 |

| pre-emergencel Gewächshaus | g/ha | Alopecurus | Amaranthus | Galium |
|---|---|---|---|---|
| Bsp. I-a-3 | 500 | 70 | 70 | 100 |

### Beispiel E

### Grenzkonzentrations-Test / Bodeninsekten - Behandlung transgener Pflanzen

### Testinsekt: Diabrotica balteata - Larven im Boden

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/1) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel F

### Heliothis virescens - Test - Behandlung transgener Pflanzen

- Lösungsmittel:: 7 Gewichtsteile Aceton
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 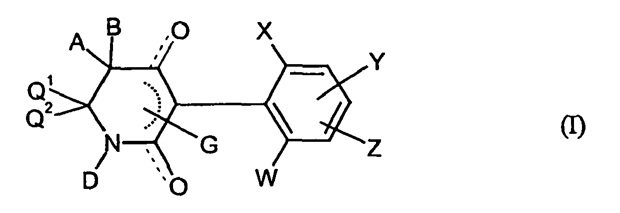 in welcher
W für Wasserstoff, Fluor, Chlor, Brom, Methyl oder Ethyl steht,
X für Chlor, Brom, Methyl, Ethyl, Propyl oder Trifluormethyl steht,
Y für Wasserstoff, Methyl, Ethyl, Fluor, Chlor, Brom oder für den Rest 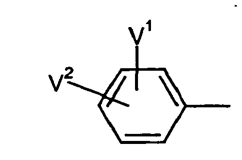 steht,
V¹ für Wasserstoff, Fluor, Chlor, Brom, Methyl, tert.-Butyl, Methoxy, Trifluormethyl oder Phenyl steht,
V² für Wasserstoff, Fluor, Chlor, Methyl, Methoxy oder Trifluormethyl steht,
Z für Wasserstoff, Chlor, Brom oder Methyl steht,
A und B für Wasserstoff stehen,
D für Wasserstoff, Methyl, Ethyl, Propyl, iso-Propyl, Cyclopropyl, Cyclopentyl oder Cyclohexyl steht,
D und Q¹ gemeinsam für Butandiyl stehen,
Q¹ für Methyl, Ethyl oder Propyl steht,
Q² für Methyl steht, wenn D und Q¹ gemeinsam für Butandiyl stehen, kann Q² auch für Wasserstoff stehen,
Q¹ und Q² gemeinsam mit dem Kohlenstoff, an den sie gebunden sind für gegebenenfalls durch Methyl, Ethyl, Methoxy, Ethoxy, Propoxy oder Butoxy substituiertes C₅-C₆-Cycloalkyl stehen, in welchem gegebe- nenfalls ein Ringglied durch Sauerstoff ersetzt ist,
G für Wasserstoff (a) oder für eine der Gruppen 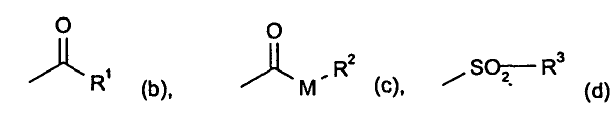 oder 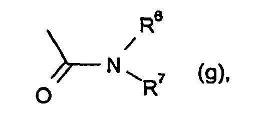 steht,
worin M für Sauerstoff oder Schwefel steht,
R¹ für C₁-C₈-Alkyl, C₂-C₄-Alkenyl, Methoxymethyl, Ethoxymethyl, Ethylthiomethyl oder gegebenenfalls durch Fluor, Chlor, Methyl, Ethyl oder Methoxy substituiertes Cyclopropyl, Cyclopentyl oder Cyclohexyl, für gegebenenfalls durch Fluor, Chlor, Brom, Cyano, Nitro, Methyl, Ethyl, iso-Propyl, tert-Butyl, Methoxy, Trifluormethyl oder Tri- fluormethoxy substituiertes Phenyl, für jeweils gegebenenfalls durch Chlor oder Methyl substituiertes Thienyl oder Pyridyl steht,
R², für jeweils gegebenenfalls durch Fluor substituiertes C₁-C₈-Alkyl, C₂-C₄-Alkenyl oder Methoxyethyl, Ethoxyethyl, Propoxyethyl, iso- Propoxyethyl, Cyclopentyl oder Cyclohexyl, oder für jeweils gegebenenfalls durch Fluor, Chlor, Cyano, Nitro, Me- thyl, Ethyl, iso-Propyl, tert-Butyl, Methoxy, Trifluormethyl oder Trifluormethoxy substituiertes Phenyl oder Benzyl steht,
R³ steht für Methyl,
R⁶ und R⁷ zusammen für einen C₆-Alkylenrest stehen, in welchem gegebenenfalls eine Methylengruppe durch Sauerstoff ersetzt ist.

2. Verfahren zur Herstellung von Verbindungen der Formel (I), **dadurch gekennzeichnet, dass** man zum Erhalt von
(A) Verbindungen der Formel (I-a) 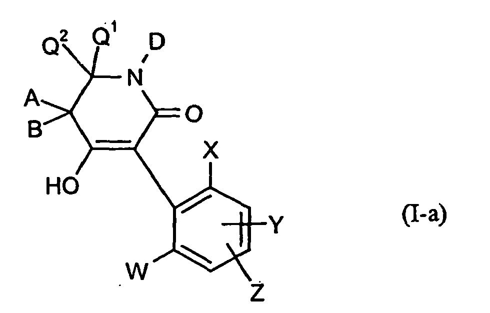 in welcher
A, B, D, Q¹, Q², W, X, Y und Z die oben angegebenen Bedeutungen haben,
Verbindungen der Formel (II) 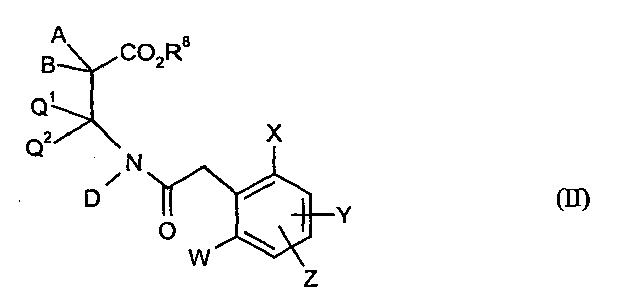 in welcher
A, B, D, Q1, Q2, W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der oben gezeigten Formeln (I-a) bis (I-g) in welchen A, B, D, G, Q¹, Q², W, X, Y und Z die oben angegebenen Bedeutungen haben,
Verbindungen der Formel (I-a') bis (I-g'), 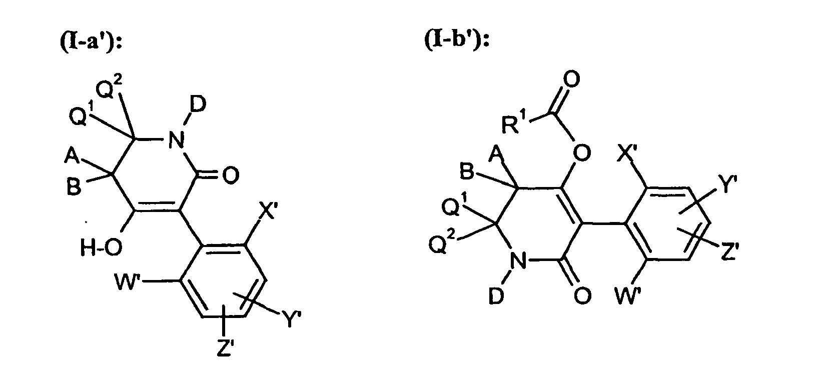 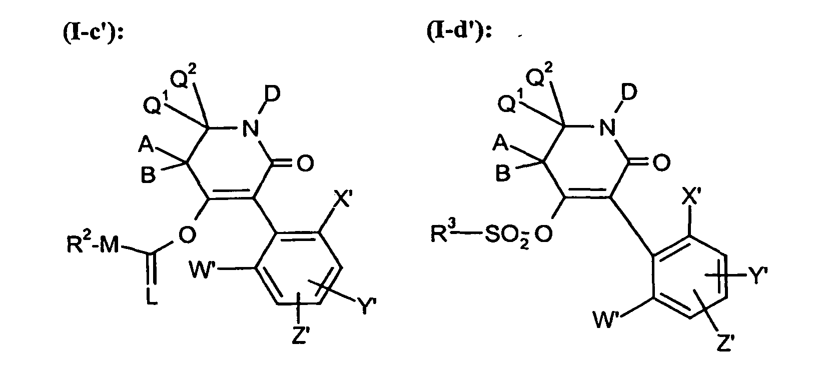 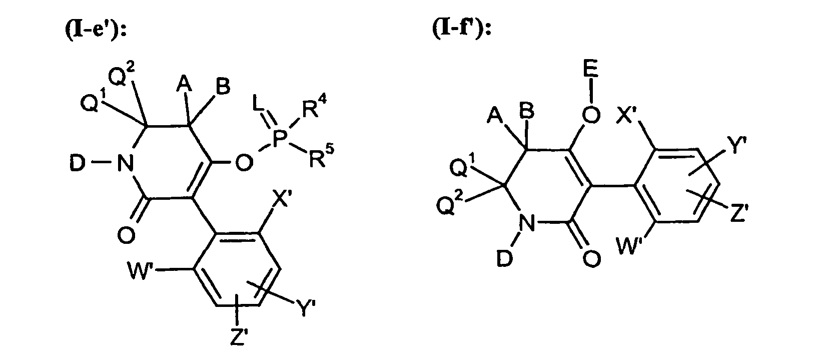 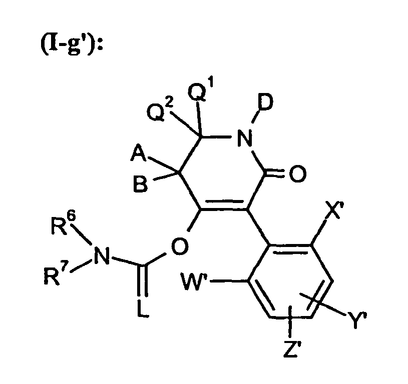 in welchen
A, B, D, R¹, R², R³, R⁴, R⁵, R⁶, R⁷, E, L, M, Q¹ Q², W', X', Y' und Z' die oben angegebene Bedeutung haben und wobei mindestens einer der Reste
W, X', Y' für Chlor, Brom oder Jod steht,
und Z' nicht für Brom oder Jod steht,
α) mit Silylacetylen der Formel (III) 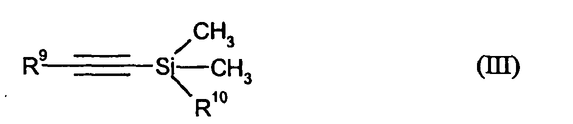 in welcher
R⁹ für Wasserstoff und
R¹⁰ für C₁-C₄-Alkyl oder Phenyl steht,
zunächst in Gegenwart eines Lösungsmittels, einer Base und eines Katalysators umsetzt, und anschließend die Silylgruppe abspaltet,
oder
B) mit Vinylstannanen der Formel (IV) 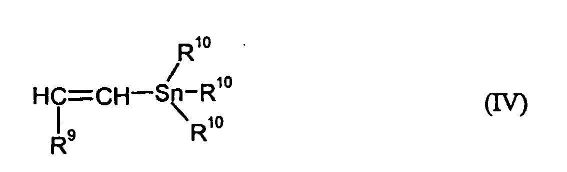 in welcher
R⁹ für Wasserstoff, Methyl oder Ethyl und
R¹⁰ für C₁-C₄-Alkyl steht,
in Gegenwart eines Lösungsmittels, gegebenenfalls in Gegenwart einer Base und in Gegenwart eines Katalysators umsetzt,
oder
γ) wenn Y' für Chlor, Brom oder Jod steht, mit Boronsäuren der Formel (V) 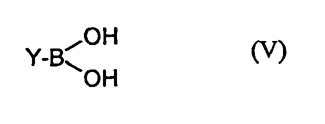 in welcher
Y für gegebenenfalls substituiertes Phenyl oder Hetaryl steht,
in Gegenwart eines Lösungsmittels, einer Base und eines Katalysators umsetzt,
(C) die Verbindungen der oben gezeigten Formel (I-b), in welcher A, B, D, Q¹, Q², R¹, W, X, Y und Z die oben angegebenen Bedeutungen haben, Verbindungen der oben gezeigten Formel (I-a), in welchen A, B, D, Q¹, Q², W, X, Y und Z die oben angegebenen Bedeutungen haben, jeweils
(α) mit Säurehalogeniden der Formel (VI) 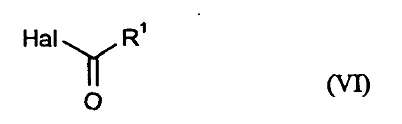 in welcher
R¹ die oben angegebene Bedeutung hat und
Hal für Halogen steht,
oder
(β) mit Carbonsäureanhydriden der Formel (VII)
R¹-CO-O-CO-R¹ (VII)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der oben gezeigten Formel (I-c), in welcher A, B, D, Q¹, Q², R², M, W, X, Y und Z die oben angegebenen Bedeutungen haben und L für Sauerstoff steht, Verbindungen der oben gezeigten Formel (I-a), in welcher A, B, D, Q¹, Q², W, X, Y und Z die oben angegebenen Bedeutungen haben, jeweils
mit Chlorameisensäureestern oder Chlorameisensäurethioestern der Formel (VIII)
R²-M-CO-Cl (VIII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(E) Verbindungen der oben gezeigten Formel (I-c), in welcher A, B, D, Q¹, Q², R², M, W, X, Y und Z die oben angegebenen Bedeutungen haben und L für Schwefel steht, Verbindungen der oben gezeigten Formel (I-a), in welcher A, B, D, Q¹, Q², W, X, Y und Z die oben angegebenen Bedeutungen haben, jeweils
mit Chlormonothioameisensäureestern oder Chlordithioameisensäureestern der Formel (IX) 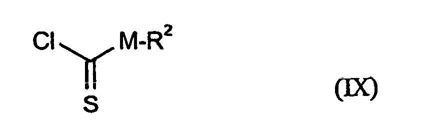 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(F) Verbindungen der oben gezeigten Formel (I-d), in welcher A, B, D, Q¹, Q², R³, W, X, Y und Z die oben angegebenen Bedeutungen haben, Verbindungen der oben gezeigten Formel (I-a), in welcher A, B, D, Q¹, Q², W, X, Y und Z die oben angegebenen Bedeutungen haben, jeweils
mit Sulfonsäurechloriden der Formel (X)
R³-SO₂-Cl (X)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der oben gezeigten Formel (I-e), in welcher A, B, D, L, Q¹, Q², R⁴, R⁵, W, X, Y und Z die oben angegebenen Bedeutungen haben, Verbindungen der oben gezeigten Formel (I-a), in welcher A, B, D, Q¹, Q², W, X, Y und Z die oben angegebenen Bedeutungen haben, jeweils
mit Phosphorverbindungen der Formel (XI) 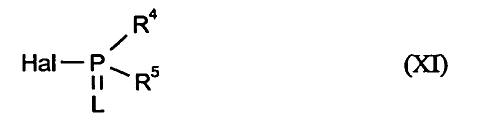 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der oben gezeigten Formel (I-f), in welcher A, B, D, E, Q¹, Q², W, X, Y und Z die oben angegebenen Bedeutungen haben, erhält, wenn man Verbindungen der oben gezeigten Formel (I-a), in welchen A, B, D, Q¹, Q², W, X, Y und Z die oben angegebenen Bedeutungen haben, jeweils
mit Metallverbindungen oder Aminen der Formeln (XII) oder (XIII)
Me(OR¹¹)ₜ (XII) 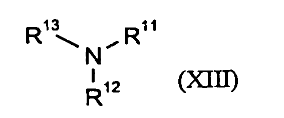
in welchen
Me für ein ein- oder zweiwertiges Metall,
t für die Zahl 1 oder 2 und
R¹¹, R¹², R¹³ unabhängig voneinander für Wasserstoff oder Alkyl stehen,
gegebenenfalls in Gegenwart eines Verdünnungsmittels umsetzt,
(I) Verbindungen der oben gezeigten Formeln (I-g), in welcher A, B, D, L, Q¹, Q², R⁶, R⁷, W, X, Y und Z die oben angegebenen Bedeutungen haben, Verbindungen der oben gezeigten Formel (I-a), in welcher A, B, D, Q¹, Q², W, X, Y und Z die oben angegebenen Bedeutungen haben, jeweils
(α) mit Isocyanaten oder Isothiocyanaten der Formel (XIV)
R⁶-N=C=L (XIV)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XV) 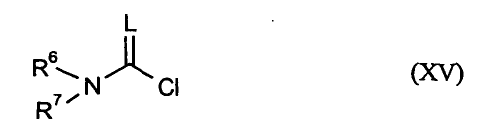 in welcher
L, R⁶ und R⁷ die oben angegebenen Bedeutungen haben, gegebenenfalls in Gegenwart eines Verdünnungsmittels und gegebenenfalls in Gegenwart eines Säurebindemittels, umsetzt.

3. Verbindungen der Formel (II) 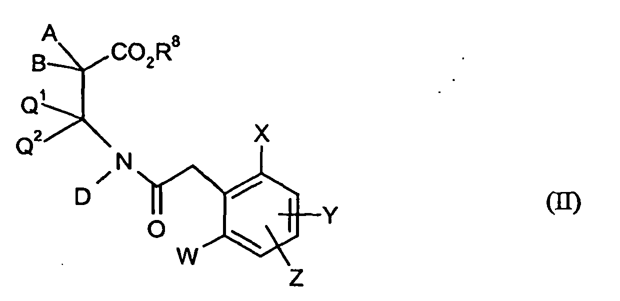 in welcher
A, B, D, Q¹, Q², W, X, Y, Z und R⁸ die oben angegebenen Bedeutungen haben,

4. Verbindungen der Formel (XVIII) 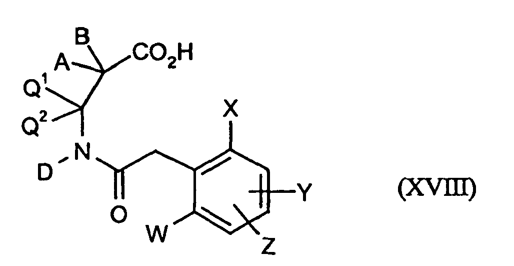 in welcher
A, B, D, Q¹, Q², W, X, Y und Z die oben angegebenen Bedeutungen haben.

5. Verbindungen der Formel (XXII) 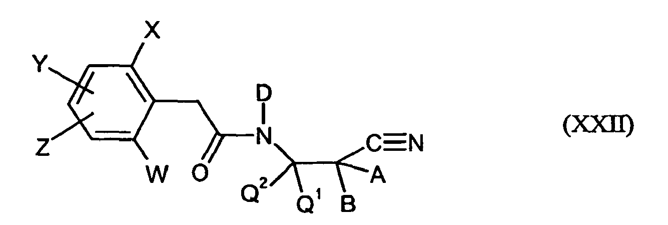 in welcher
A, B, D, Q¹, Q², W, X, Y und Z die oben angegebenen Bedeutungen haben.

6. Schädlingsbekämpfungsmittel und Herbizide, **gekennzeichnet durch** einen Gehalt an mindestens einer Verbindung der Formel (I) gemäß Anspruch 1.

7. Verfahren zur Bekämpfung von tierischen Schädlingen und unerwünschten Pflanzenbewuchs, **dadurch gekennzeichnet, dass** man Verbindungen der Formel (I) gemäß Anspruch 1 auf Schädlinge und/oder ihren Lebensraum einwirken lässt, aüsgenommen am menschlichen oder tierischen Körper.

8. Verwendung von Verbindungen der Formel (I) gemäß Anspruch 1 zur Bekämpfung von tierischen Schädlingen und unerwünschtem Pflanzenbewuchs, ausgenommen am menschlichen oder tierischen Körper.

9. Verfahren zur Herstellung von Schädlingsbekämpfungsmitteln und Herbiziden, **dadurch gekennzeichnet, dass** man Verbindungen der Formel (I) gemäß Anspruch 1 mit Streckmitteln und/oder oberflächenaktiven Stoffen vermischt.

10. Verwendung von Verbindungen der Formel (I) gemäß Anspruch 1 zur Herstellung von Schädlingsbekämpfungsmitteln und Herbiziden.

## Claims

1. Compounds of the formula (I) 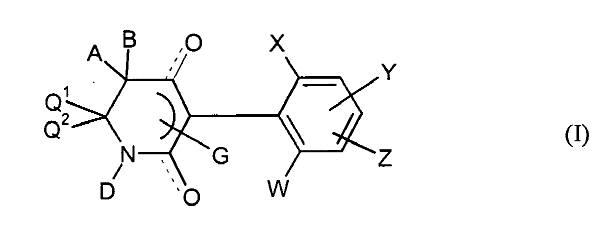 in which
W represents hydrogen, fluorine, chlorine, bromine, methyl or ethyl,
X represents chlorine, bromine, methyl, ethyl, propyl or trifluoromethyl,
Y represents hydrogen, methyl, ethyl, fluorine, chlorine, bromine or represents the radical 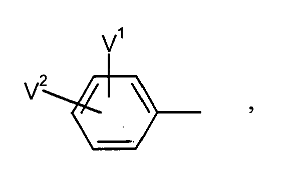
V¹ represents hydrogen, fluorine, chlorine, bromine, methyl, tert- butyl, methoxy, trifluoromethyl or phenyl,
V² represents hydrogen, fluorine, chlorine methyl, methoxy or trifluoromethyl,
Z represents hydrogen, chlorine, bromine or methyl,
A and B represent hydrogen,
D represents hydrogen, methyl, ethyl, propyl, iso-propyl, cyclopropyl, cyclopentyl or cyclohexyl,
D and Q¹ together represent butanediyl,
Q¹ represents methyl, ethyl or propyl,
Q² represents methyl; if D and Q¹ together represent butanediyl, Q² may also represent hydrogen,
Q¹ and together with the carbon to which they are attached represent optionally methyl-, ethyl-, methoxy-, ethoxy-, propoxy- or butoxy- substituted C₅-C₆-cycloalkyl in which optionally one ring member is replaced by oxygen,
G represents hydrogen (a) or represents one of the groups 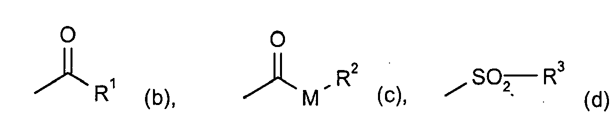 or 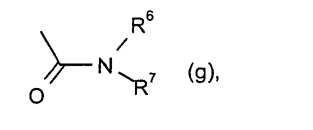 in which M represents oxygen or sulphur,
R¹ represents C₁-C₈-alkyl, C₂-C₄-alkenyl, methoxymethyl, ethoxymethyl, ethylthiomethyl or optionally fluorine-, chlorine-, methyl-, ethyl- or methoxy-substituted cyclopropyl, cyclopentyl or cyclohexyl, represents optionally fluorine-, chlorine-, bromine-, cyano-, nitro-, methyl-, ethyl-, iso-propyl-, tert-butyl-, methoxy-, trifluoromethyl- or trifluoromethoxy-substituted phenyl, represents in each case optionally chlorine- or methyl-substituted thienyl or pyridyl,
R² represents in each case optionally fluorine-substituted C₁-C₈-alkyl, C₂-C₄-alkenyl or methoxyethyl, ethoxyethyl, propoxyethyl, iso- propoxyethyl, cyclopentyl or cyclohexyl, or represents in each case optionally fluorine-, chlorine-, cyano-, nitro-, methyl-, ethyl-, iso-propyl-, tert-butyl-, methoxy-, trifluoromethyl- or trifluoromethoxy-substituted phenyl or benzyl,
R³ represents methyl,
R⁶ and R⁷ together represent a C₆-alkylene radical in which optionally one methylene group is replaced by oxygen.

2. Process for preparing compounds of the formula (I), **characterized in that** to obtain
(A) compounds of the formula (I-a) 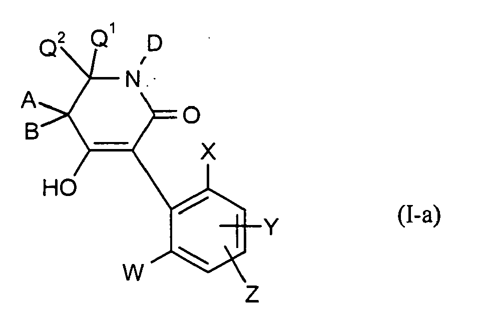 in which
A, B, D, Q¹, Q², W, X, Y and Z are each as defined above,
compounds of the formula (II) 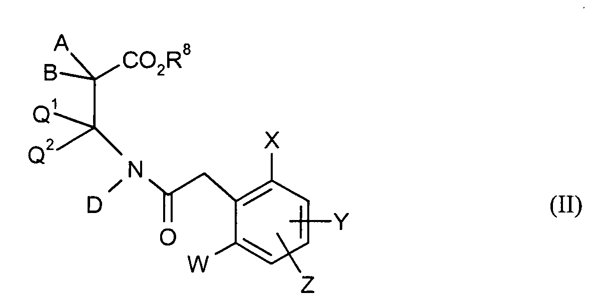 in which
A, B, D, Q¹, Q², W, X, Y and Z are each as defined above,
and
R⁸ represents alkyl,
are condensed intramolecularly in the presence of a diluent and in the presence of a base,
(B) compounds of the formulae (I-a) to (I-g) shown above in which A, B, D, G, Q¹, Q², W, X, Y and Z are each as defined above,
compounds of the formulae (I-a') to (I-g'), 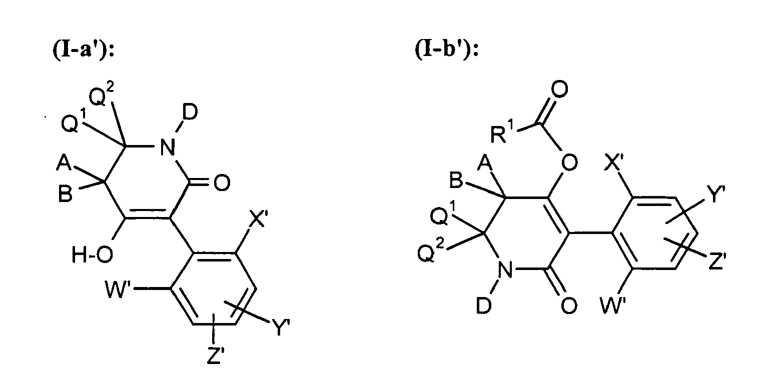 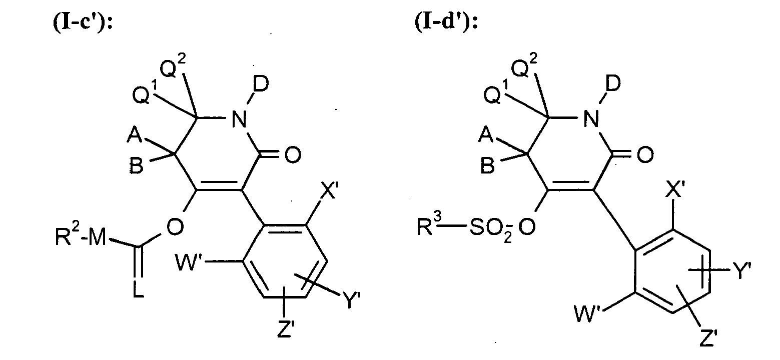 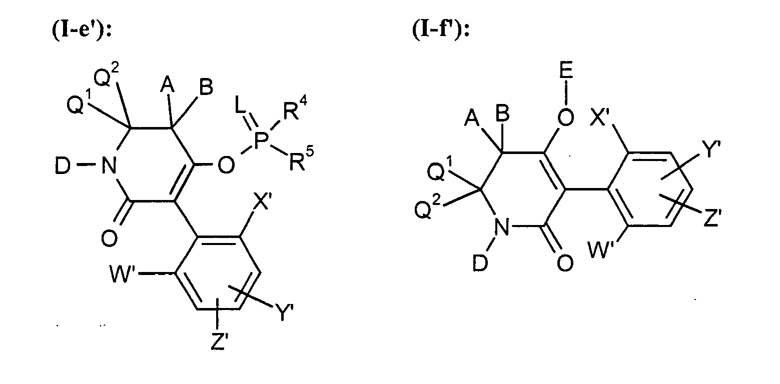 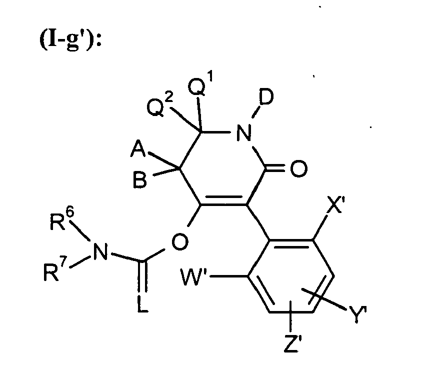 in which
A, B, D, R¹, R², R³, R⁴, R⁵, R⁶, R⁷, E, L, M, Q¹, Q², W', X', Y' and Z'
are each as defined above and where at least one of the radicals
W', X', Y' represents chlorine, bromine or iodine
and Z' does not represent bromine or iodine
α) are initially reacted with silylacetylene of the formula (III) 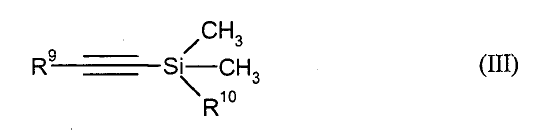 in which
R⁹ represents hydrogen and
R¹⁰ represents C₁-C₄-alkyl or phenyl,
in the presence of a solvent, a base and a catalyst, and the silyl group is then removed,
or
ß) are reacted with vinylstannanes of the formula (IV) 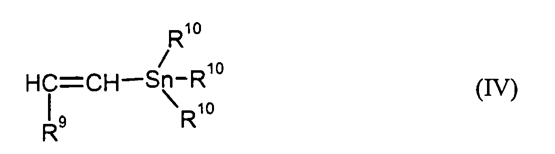 in which
R⁹ represents hydrogen, methyl or ethyl and
R¹⁰ represents C₁-C₄-alkyl,
in the presence of a solvent, if appropriate in the presence of a base and in the presence of a catalyst,
or
γ) if Y' represents chlorine, bromine or iodine, are reacted with boronic acids of the formula (V) 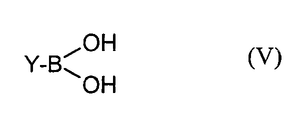 in which
Y represents optionally substituted phenyl or hetaryl,
in the presence of a solvent, a base and a catalyst,
(C) the compounds of the formula (I-b) shown above in which A, B, D, Q¹, Q², R¹, W, X, Y and Z are each as defined above, compounds of the formula (I-a) shown above in which A, B, D, Q¹, Q², W, X, Y and Z are each as defined above are in each case reacted
(α) with acid halides of the formula (VI) 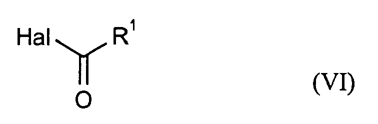 in which
R¹ is as defined above and
Hal represents halogen,
or
(β) with carboxylic anhydrides of the formula (VII)
R¹-CO-O-CO-R¹ (VII)
in which
R¹ is as defined above,
if appropriate in the presence of a diluent and if appropriate in the presence of an acid binder;
(D) compounds of the formula (I-c) shown above in which A, B, D, Q¹, Q², R², M, W, X, Y and Z are each as defined above and L represents oxygen, compounds of the formula (I-a) shown above in which A, B, D, Q¹, Q², W, X, Y and Z are each as defined above are in each case reacted with chloroformic esters or chloroformic thioesters of the formula (VIII)
R²-M-CO-Cl (VIII)
in which
R² and M are each as defined above
if appropriate in the presence of a diluent and if appropriate in the presence of an acid binder;
(E) compounds of the formula (I-c) shown above in which A, B, D, Q¹, Q², R², M, W, X, Y and Z are each as defined above and L represents sulphur, compounds of the formula (I-a) shown above in which A, B, D, Q¹, Q², W, X, Y and Z are each as defined above are in each case reacted
with chloromonothioformic esters or chlorodithioformic esters of the formula (IX) 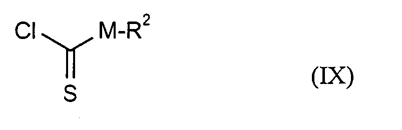 in which
M and R² are each as defined above
if appropriate in the presence of a diluent and if appropriate in the presence of an acid binder,
and
(F) compounds of the formula (I-d) shown above in which A, B, D, Q¹, Q², R³, W, X, Y and Z are each as defined above, compounds of the formula (I-a) shown above in which A, B, D, Q¹, Q², W, X, Y and Z are each as defined above are in each case reacted
with sulphonyl chlorides of the formula (X)
R³-SO₂-Cl (X)
in which
R³ is as defined above,
if appropriate in the presence of a diluent and if appropriate in the presence of an acid binder,
(G) compounds of the formula (I-e) shown above in which A, B, D, L, Q¹, Q², R⁴, R⁵, W, X, Y and Z are each as defined above, compounds of the formula (I-a) shown above in which A, B, D, Q¹, Q², W, X, Y and Z are each as defined above are in each case reacted
with phosphorus compounds of the formula (XI) 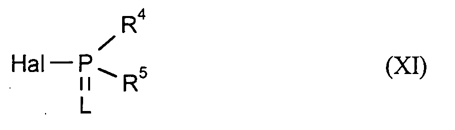 in which
L, R⁴ and R⁵ are each as defined above and
Hal represents halogen,
if appropriate in the presence of a diluent and if appropriate in the presence of an acid binder,
(H) compounds of the formula (I-f) shown above in which A, B, D, E, Q¹, Q², W, X, Y and Z are each as defined above, compounds of the formula (I-a) shown above in which A, B, D, Q¹, Q², W, X, Y and Z are each as defined above are in each case reacted
with metal compounds or amines of the formulae (XII) and (XIII)
Me(OR¹¹)ₜ (XII) 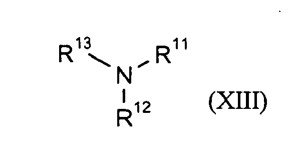
in which
Me represents a mono- or divalent metal,
t represents the number 1 or 2 and
R¹¹, R¹², R¹³ independently of one another each represent hydrogen or alkyl,
if appropriate in the presence of a diluent,
(I) compounds of the formula (I-g) shown above in which A, B, D, L, Q¹, Q², R⁶, R⁷, W, X, Y and Z are each as defined above, compounds of the formula (I-a) shown above in which A, B, D, Q¹, Q², W, X, Y and Z are each as defined above are in each case reacted
(α) with isocyanates or isothiocyanates of the formula (XIV)
R⁶-N=C=L (XIV)
in which
R⁶ and L are each as defined above
if appropriate in the presence of a diluent and if appropriate in the presence of a catalyst, or
(β) with carbamoyl chlorides or thiocarbamoyl chlorides of the formula (XV) 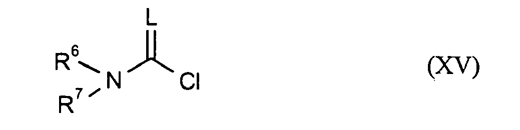 in which
L, R⁶ and R⁷ are each as defined above,
if appropriate in the presence of a diluent and if appropriate in the presence of an acid binder.

3. Compounds of the formula (II) 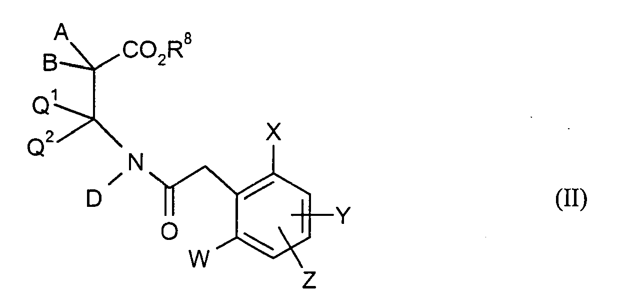 in which
A, B, D, Q¹, Q², W, X, Y, Z and R⁸ are each as defined above.

4. Compounds of the formula (XVIII) 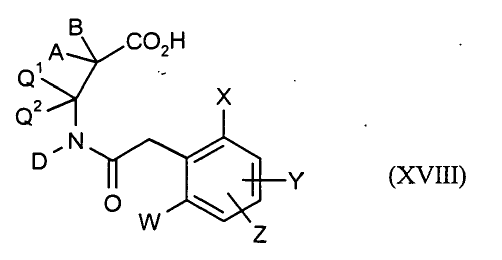 in which
A, B, D, Q¹, Q², W, X, Y and Z are each as defined above.

5. Compounds of the formula (XXII) 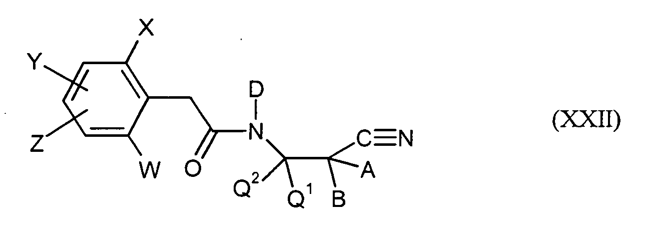 in which
A, B, D, Q¹, Q², W, X, Y and Z are each as defined above.

6. Pesticides and herbicides, **characterized in that** they comprise at least one compound of the formula (I) according to Claim 1.

7. Method for controlling animal pests and undesirable vegetation, **characterized in that** compounds of the formula (I) according to Claim 1 are allowed to act on pests and/or their habitat, except or on the human or animal body.

8. Use of compounds of the formula (I) according to Claim 1 for controlling animal pests and undesirable vegetation, except for on the human or animal body.

9. Process for preparing pesticides and herbicides, **characterized in that** compounds of the formula (I) according to Claim 1 are mixed with extenders and/or surfactants.

10. Use of compounds of the formula (I) according to Claim 1 for preparing pesticides and herbicides.

## Revendications

1. Composés de formule (I) 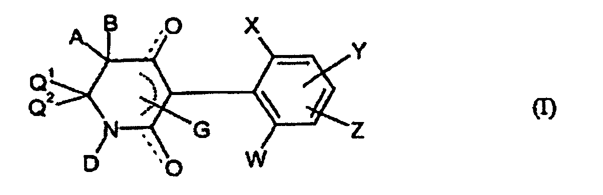 dans laquelle
W représente l'hydrogène, le fluor, le chlore, le brome, un reste méthyle ou éthyle,
X représente le chlore, le brome, un reste méthyle, éthyle, propyle ou trifluorométhyle,
Y représente l'hydrogène, un reste méthyle, éthyle, le fluor, le chlore, le brome ou le reste 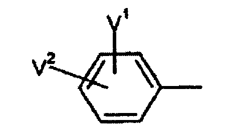
V¹ représente l'hydrogène, le fluor, le chlore, le brome, un reste méthyle, tertiobutyle, méthoxy, trifluorométhyle ou phényle,
V² représente l'hydrogène, le fluor, le chlore, un reste méthyle, méthoxy ou trifluorométhyle,
Z représente l'hydrogène, le chlore, le brome ou un reste méthyle,
A et B représente l'hydrogène,
D représente l'hydrogène, un reste méthyle, éthyle, propyle, isopropyle, cyclopropyle, cyclopentyle ou cyclohexyle,
D et Q¹ représentent conjointement un reste butanediyle,
Q¹ représente un reste méthyle, éthyle ou propyle,
Q² représente un reste méthyle, Q² pouvant aussi représenter l'hydrogène lorsque D et Q¹ forment conjointement un reste butanediyle,
Q¹ et Q² forment conjointement avec l'hydrogène auquel ils sont liés un reste cycloalkyle en C₅ ou C₆ éventuellement substitué par un radical méthyle, éthyle, méthoxy, éthoxy, propoxy ou butoxy, et dans lequel le cas échéant un chaînon du noyau est remplacé par l'oxygène,
G représente l'hydrogène (a) ou l'un des groupes 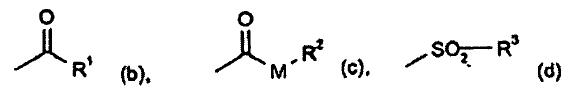 ou 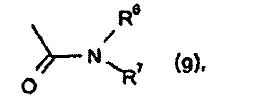 où M représente l'oxygène ou le soufre,
R¹ représente un reste alkyle en C₁ à C₈, alcényle en C₂ à C₄, méthoxyméthyle, éthoxyméthyle, éthyl- thiométhyle ou un reste cyclohexyle, cyclopentyle ou cyclopropyle éventuellement substitué par un radical fluoro, chloro, méthyle, éthyle ou méthoxy, un reste phényle éventuellement substitué par un radical fluoro, chloro, bromo, cyano, nitro, méthyle, éthyle, isopropyle, tertiobutyle, méthoxy, trifluorométhyle ou trifluorométhoxy, un reste thiényle ou pyridyle, chacun éventuellement substitué par un radical chloro ou méthyle,
R² représente un reste, éventuellement substitué dans chaque cas par du fluor, alkyle en C₁ à C₈, alcényle en C₂ à C₄ ou méthoxyéthyle, éthoxy- éthyle, propoxyéthyle, isopropoxyéthyle, cyclopentyle ou cyclohexyle, ou bien un reste phényle ou benzyle, chacun éventuellement substitué par un radical fluoro, chloro, cyano, nitro, méthyle, éthyle, isopropyle, tertiobutyle, méthoxy, trifluorométhyle ou trifluorométhoxy,
R³ représente un reste méthyle,
R⁶ et R⁷ forment conjointement un reste alkylène en C₆ dans lequel, le cas échéant, un groupe méthylène est remplacé par de l'oxygène.

2. Procédé de production de composés de formule (I), **caractérisé en ce que**
(A) pour obtenir des composés de formule (I-a) 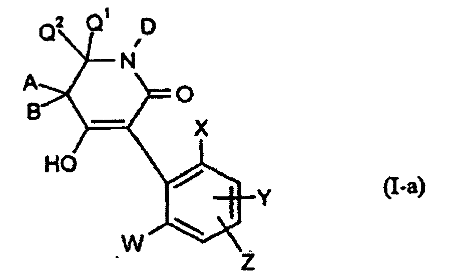 dans laquelle
A, B, D, Q¹, Q², W, X, Y et Z ont les définitions indiquées ci-dessus,
des composés de formule (II) 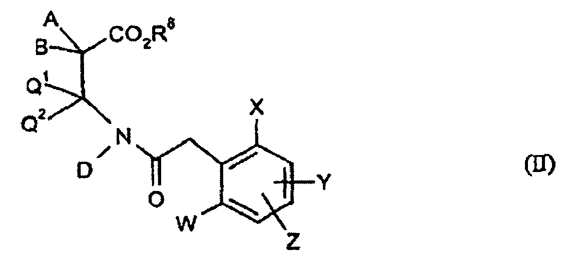 dans laquelle
A, B, D, Q¹, Q², W, X, Y et Z ont les définitions indiquées ci-dessus,
et
R⁸ représente un reste alkyle,
sont soumis à une condensation intramoléculaire en présence d'un diluant et en présence d'une base,
(B) pour obtenir des composés des formules (I-a) à (I-g) représentées ci-dessus,
dans lesquelles A, B, D, G, Q¹, Q², W, X, Y et Z ont les définitions indiquées ci-dessus, des composés de formules (I-a') à (I-g') 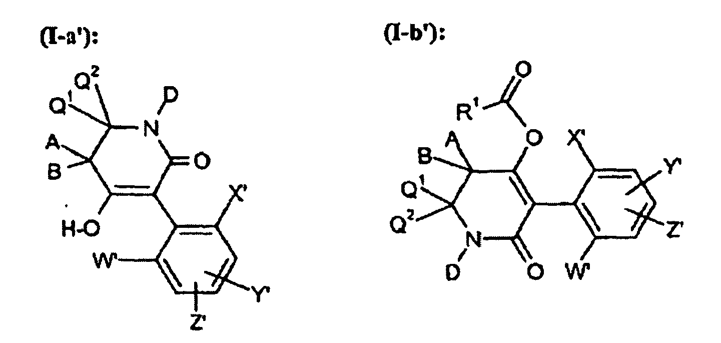 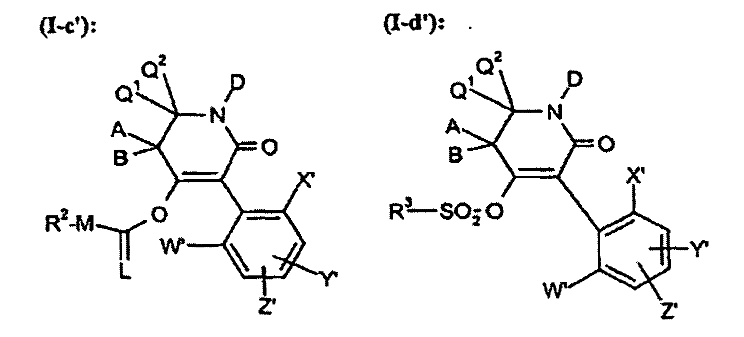 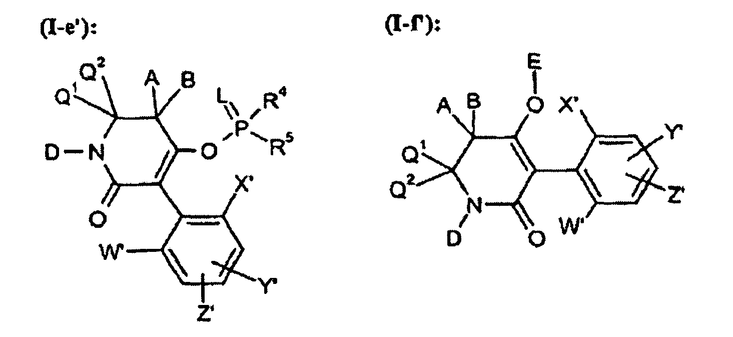 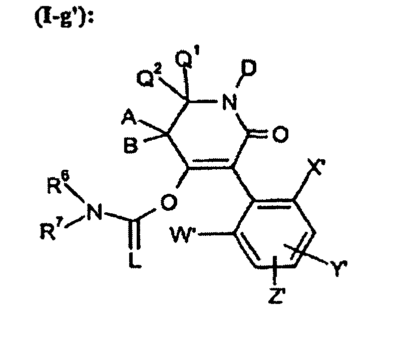 dans lesquelles
A, B, D, R¹, R² R³, R⁴, R⁵, R⁶, R⁷, E, L, M, Q¹, Q², W', X', Y' et Z' ont les définitions indiquées ci-dessus et où au moins l'un des restes
W', X', Y' représente le chlore, le brome ou l'iode, et Z' ne représente pas le brome ou l'iode,
α) sont tout d'abord amenés à réagir avec un silylacétylène de formule (III) 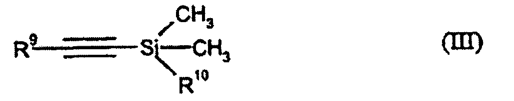 dans laquelle
R⁹ représente l'hydrogène et
R¹⁰ représente un reste alkyle en C₁ à C₄ ou phényle,
en présence d'un solvant, d'une base et d'un catalyseur, puis le groupe silyle est éliminé,
ou bien
P) sont amenés à réagir avec des vinylstannanes de formule (IV) 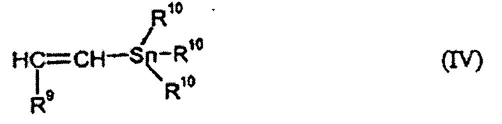 dans laquelle
R⁹ représente l'hydrogène, un reste méthyle ou éthyle, et
R¹⁰ représente un reste alkyle en C₁ à C₄,
en présence d'un solvant, éventuellement en présence d'une base et en présence d'un catalyseur,
ou bien
γ) sont amenés à réagir, lorsque Y' représente le chlore, le brome ou l'iode, avec des acides boroniques de formule (V) 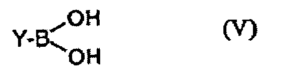 dans laquelle
Y représente un reste hétaryle ou phényle éventuellement substitué,
en présence d'un solvant, d'une base et d'un catalyseur,
(C) pour obtenir les composés de formule (I-b) représentée ci-dessus, dans laquelle A, B, D, Q¹, Q², R¹, W, X, Y et Z ont des définitions indiquées ci-dessus, des composés de composés de formule (I-a) représentée ci-dessus, dans laquelle A, B, D, Q¹, Q², W, X, Y et Z ont les définitions indiquées ci-dessus, sont dans chaque cas amenés à réagir
(α) avec des halogénures d'acides de formule (VI) 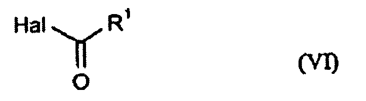 dans laquelle
R¹ a la définition indiquée ci-dessus et
Hal représente un halogène,
ou bien
(β) avec des anhydrides d'acides carboxyliques de formule (VII)
R¹-CO-Q-CO-R¹ (VII)
dans laquelle
R¹ a la définition indiquée ci-dessus,
éventuellement en présence d'un diluant et, le cas échéant, en présence d'un accepteur d'acide ;
(D) pour obtenir des composés de formule (I-c) représentée ci-dessus, dans laquelle A, B, D, Q¹, Q², R², M, W, X, Y et Z ont les définitions indiquées ci-dessus et L représente l'oxygène, des composés de formule (I-a) représentée ci-dessus dans laquelle A, B, D, Q¹, Q², W, X, Y et Z ont les définitions indiquées ci-dessus, sont dans chaque cas amenés à réagir
avec des esters d'acide chloroformique ou des thioesters d'acide chloroformique de formule (VIII)
R²-M-CO-CI (VIII)
dans laquelle
R² et M ont les définitions indiquées ci-dessus,
éventuellement en présence d'un diluant et, le cas échéant, en présence d'un accepteur d'acide ;
(E) pour obtenir des composés de formule (I-c) représentée ci-dessus, dans laquelle A, B, D, Q¹, Q², R², M, W, X, Y et Z ont les définitions indiquées ci-dessus et L représente le soufre, des composés de formule (I-a) représentée ci-dessus, dans laquelle A, B, D, Q¹, Q², W, X, Y et Z ont les définitions indiquées ci-dessus, sont dans chaque cas amenés à réagir
avec des esters d'acide chloromonothioformique ou des esters d'acide chlorodithioformique de formule (IX) 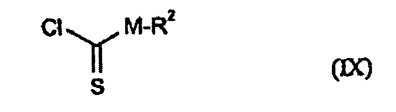 dans laquelle
M et R² ont les définitions indiqués ci-dessus, éventuellement en présence d'un diluant et, le cas échéant, en présence d'un accepteur d'acide
et
(F) pour obtenir des composés de formule (I-d) représentée ci-dessus, dans laquelle A, B, D, Q¹, Q², R³, W, X, Y et Z ont les définitions indiquées ci-dessus, des composés de formule (I-a) représentée ci-dessus, dans laquelle A, B, D, Q¹, Q², W, X, Y et Z ont les définitions indiquées ci-dessus, sont dans chaque cas amenés à réagir
avec des chlorures d'acides sulfoniques de formule (X)
R³-SO₂-Cl (X)
dans laquelle
R³ a la définition indiquée ci-dessus,
éventuellement en présence d'un diluant et, le cas échéant, en présence d'un accepteur d'acide,
(G) pour obtenir des composés de formule (I-e) représentée ci-dessus dans laquelle A, B, D, L, Q¹, Q², R⁴, R⁵, W, X, Y et Z ont les définitions indiquées ci-dessus, des composés de formule (I-a) représentée ci-dessus dans laquelle A, B, D, Q¹, Q², W, X, Y et Z ont les définitions indiquées ci-dessus, sont dans chaque cas amenés à réagir
avec des composés de phosphore de formule (XI) 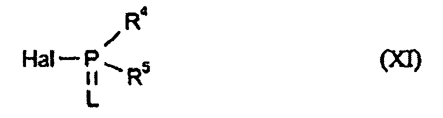 dans laquelle
L, R⁴ et R⁵ ont les définitions indiquées ci-dessus et Hal représente un halogène
éventuellement en présence d'un diluant et, le cas échéant, en présence d'un accepteur d'acide,
(H) pour obtenir des composés de formule (I-f) représentée ci-dessus, dans laquelle A, B, D, E, Q¹, Q², W, X, Y et Z ont les définitions indiquées ci-dessus, des composés de formule (I-a) représentée ci-dessus dans laquelle A, B, D, Q¹, Q², W, X, Y et Z ont les définitions indiquées ci-dessus, sont dans chaque cas amenés à réagir
avec des composés métalliques ou des amines de formules (XII) ou (XIII)
Me(OR¹¹)ₜ (XII) 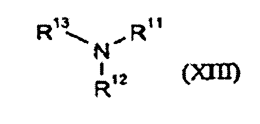
dans lesquelles
Me représente un métal monovalent ou divalent,
t représente le nombre 1 ou 2 ou
R¹¹, R¹² et R¹³ représentent, indépendamment les uns des autres, l'hydrogène ou un reste alkyle,
éventuellement en présence d'un diluant,
(I) pour obtenir des composés de formule (I-g) représentée ci-dessus, dans laquelle A, B, D, L, Q¹, Q², R⁶, R⁷, W, X, Y et Z ont les définitions indiquées ci-dessus, des composés de formule (I-a) représentée ci-dessus, dans laquelle A, B, D, Q¹, Q², W, X, Y et Z ont les définitions indiquées ci-dessus, sont dans chaque cas amenés à régir
(α) avec des isocyanates ou des isothiocyanates de formule (XIV)
R⁶-N=C=L (XIV)
dans laquelle
R⁶ et L ont les définitions indiquées ci-dessus, éventuellement en présence d'un diluant et, le cas échéant, en présence d'un catalyseur, ou bien
(β) avec des chlorures d'acides carbamiques ou des chlorures d'acides thiocarbamiques de formule (XV) 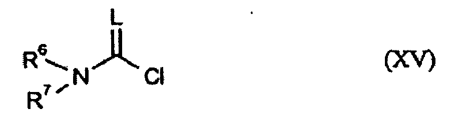 dans laquelle
L, R⁶ et R⁷ ont les définitions indiquées ci-dessus, éventuellement en présence d'un diluant et, le cas échéant, en présence d'un accepteur d'acide.

3. Composés de formule (II) 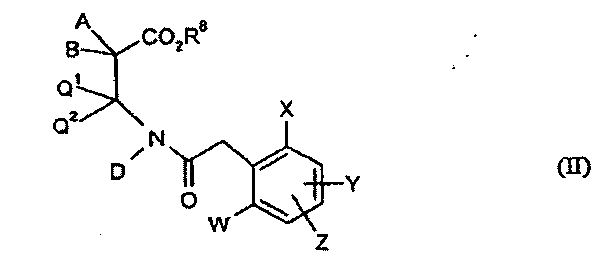 dans laquelle
A, B, D, Q¹, Q², W, X, Y, Z et R⁸ ont les définitions indiquées ci-dessus.

4. Composés de formule (XVIII) 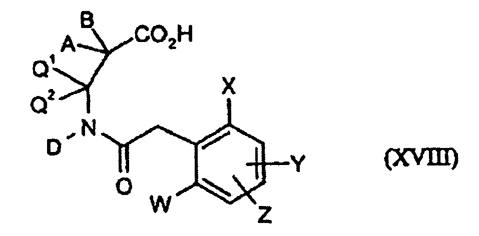 dans laquelle
A, B, D, Q¹, Q², W, X, Y et Z ont les définitions indiquées ci-dessus.

5. Composés de formule (XXII) 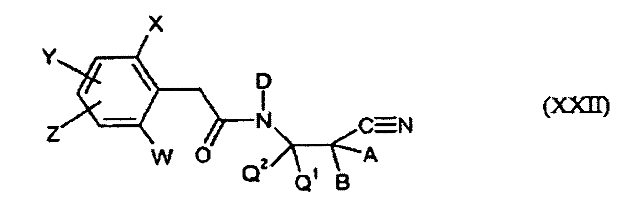 dans laquelle
A, B, D, Q¹, Q², W, X, Y et Z ont les définitions indiquées ci-dessus.

6. Compositions pesticides et herbicides, **caractérisés par** une teneur en au moins un composé de formule (I) suivant la revendication 1.

7. Procédé pour combattre des parasites animaux et une végétation indésirable, **caractérisé en ce qu'**on fait agir des composés de formule (I) suivant la revendication 1 sur des parasites et/ou sur leur milieu, excepté par application au corps humain ou animal.

8. Utilisation de composés de formule (I) suivant la revendication 1, pour combattre des parasites animaux et une végétation indésirable, excepté par application au corps humain ou animal.

9. Procédé de préparation de compositions pesticides et herbicides, **caractérisé en ce qu'**on mélange des composés de formule (I) suivant la revendication 1 avec des diluants et/ou des agents tensioactifs.

10. Utilisation de composés de formule (I) suivant la revendication 1 pour la préparation de compositions pesticides et herbicides.
